# EUROPEAN PATENT APPLICATION

(11) **EP 3 299 450 A1**
(43) Date of publication of application: **28.03.2018**
(21) Application number: 16796471.7
(22) Date of filing: 16.05.2016
(51) Int. Cl.: C12N 5/07, C12N 5/10, C12Q 1/02, G01N 33/15, G01N 33/50, C12N 15/09

(54) **INDUCED SYNCYTIOTROPHOBLAST AND PREPARATION METHOD FOR PROGENITOR CELL THEREOF**

(30) Priority: 18.05.2015 JP 2015100804
(71) Applicant: Sumitomo Chemical Company, Ltd., Chuo-ku Tokyo 104-8260 (JP)
(72) Inventor: ASANO, Kouji, Osaka-shi Osaka 554-8558 (JP); SAITO, Koichi, Osaka-shi Osaka 554-8558 (JP); MAHARA, Tomo, Osaka-shi, Osaka 554-8558 (JP)
(74) Representative: J A Kemp
(86) International application number: PCT/JP2016/064494
(87) International publication number: WO 2016/186078

(57) **Abstract**

According to the present invention, there is provided a method for producing artificial trophoblasts derived from human cells, that includes the steps of: adhesion culturing human pluripotent stem cells in a culture medium containing a BMP signal transduction activator, and during the culturing bringing the cells under culturing into contact with at least one selected from the group consisting of a γ aminobutyric acid B receptor activator, a peroxisome proliferator-activated receptor γ activator, a retinoid X receptor activator, and a retinoic acid receptor activator, thereby obtaining a culture containing trophoblasts differentiated from the human pluripotent stem cells, and so on.

SEQUENCE LISTING

<110> Sumitomo Chemical Co., Ltd.

<120> Methods for producing artificial syncytiotrophoblast and progenitor
cell thereof

<130> S40085WO01

<150> JP2015-100804
<151> 2015-05-18

<160> 1

<210> 1

<211> 36

<212> DNA

<213> Homo sapiens

<400> 1
tggcctgaccttatcatggaacctgacgactttgat                                    36

## Description

### TECHNICAL FIELD

The present invention relates to methods for producing artificial syncytiotrophoblasts and progenitor cells thereof from human pluripotent stem cells.

### BACKGROUND ART

A substance that is taken in a pregnant woman is exposed to a fetus after it permeates the placenta. Since whether a substance permeates the placenta or not largely influences on the expression of the developmental toxicity to the fetus by the substance, there is a demand for development of an in vitro test system for evaluating the human placenta permeability of a substance.

Syncytiotrophoblasts form a cell layer that is located at the outermost side in the placental villus and contacts the maternal blood. In the placental villus, cytotrophoblasts that exist inside syncytiotrophoblasts are differentiated and fused to form syncytiotrophoblasts.

Syncytiotrophoblasts form a blood placenta barrier together with cytotrophoblasts, vascular endothelium and so on. Syncytiotrophoblasts exists at the outermost side in the blood placenta barrier and contacts the maternal blood. Syncytiotrophoblasts form tight junction between cells and limit the diffusive permeation at the blood placenta barrier. In syncytiotrophoblasts, various efflux transporters (for example, MDR1) and uptake transporters (for example, GLUT1) are expressed, and selective efflux and uptake of substances are performed via these transporters. Thus, syncytiotrophoblasts play a principal role in control at the blood placenta barrier in the transfer of substances between the mother and the fetus.

A method of culturing human embryonic stem cells in a culture medium containing a BMP signal transduction activator such as bone morphogenetic protein (hereinafter, sometimes referred to as BMP) 4 to thereby induce differentiation into trophoblasts has been known (Patent Document 1). There has been reported that when human embryonic stem cells were cultured in a culture medium containing BMP4, a fibroblast growth factor (hereinafter, sometimes referred to as FGF) signal transduction inhibitor and an activin signal transduction inhibitor, differentiation into syncytiotrophoblasts was induced (Non-patent Document 1). There has been reported that when human embryonic stem cells were cultured in a culture medium containing BMP4, an FGF2 signal transduction inhibitor and an activin signal transduction inhibitor, syncytiotrophoblast-like cells and extravillous trophoblast-like cells appeared (Non-patent Document 2).

### PRIOR ART DOCUMENTS

### PATENT DOCUMENT

Patent Document 1: WO2003/078599

### NON-PATENT DOCUMENTS

Non-Patent Document 1: STEM CELLS AND DEVELOPMENT, Volume: 21, Pages: 2987-3000(2012)
Non-Patent Document 2: Proceedings of the National Academy of Sciences, Volume: 110, Pages: 1212-1221(2013)

### SUMMARY OF THE INVENTION

### PROBLEMS TO BE SOLVED BY THE INVENTION

There has been demanded for development of a method for producing artificial syncytiotrophoblasts derived from human cells, and progenitor cells thereof in vitro with high efficiency.

### MEANS FOR SOLVING THE PROBLEMS

The present invention provides methods for producing artificial syncytiotrophoblasts and progenitor cells thereof from human pluripotent stem cells.

Specifically, the present invention provides:
Item 1. A method for producing artificial trophoblasts derived from human cells, comprising the steps of: adhesion culturing human pluripotent stem cells in a culture medium containing a BMP signal transduction activator, and during the culturing bringing the cells under culturing into contact with at least one selected from the group consisting of a γ aminobutyric acid B receptor activator, a peroxisome proliferator-activated receptor γ activator, a retinoid X receptor activator, and a retinoic acid receptor activator, thereby obtaining a culture containing trophoblasts differentiated from the human pluripotent stem cells (hereinafter, sometimes referred to as Production method 1 of the present invention);
Item 2. A method for producing artificial syncytiotrophoblasts derived from human cells, comprising the steps of: adhesion culturing human pluripotent stem cells in a culture medium containing a BMP signal transduction activator, and during the culturing bringing the cells under culturing into contact with at least one selected from the group consisting of a γ aminobutyric acid B receptor activator, a peroxisome proliferator-activated receptor γ activator, a retinoid X receptor activator, and a retinoic acid receptor activator, thereby obtaining a culture containing syncytiotrophoblasts differentiated from the human pluripotent stem cells (hereinafter, sometimes referred to as Production method 2 of the present invention);
Item 3. The production method according to item 1 or 2, wherein the contact of the cells under culturing with at least one selected from the group consisting of a γ aminobutyric acid B receptor activator, a peroxisome proliferator-activated receptor γ activator, a retinoid X receptor activator, and a retinoic acid receptor activator is started before cells expressing GCM1 mRNA appear in the culture;
Item 4. The production method according to any one of items 1 to 3, wherein the contact of the cells under culturing with at least one selected from the group consisting of a γ aminobutyric acid B receptor activator, a peroxisome proliferator-activated receptor γ activator, a retinoid X receptor activator, and a retinoic acid receptor activator is conducted from before cells expressing GCM1 mRNA appear in the culture to after cells expressing GCM1 mRNA appear;
Item 5. The production method according to any one of items 1 to 4, wherein the contact of the cells under culturing with at least one selected from the group consisting of a γ aminobutyric acid B receptor activator, a peroxisome proliferator-activated receptor γ activator, a retinoid X receptor activator, and a retinoic acid receptor activator is conducted over the entire period of the culturing in the culture medium containing a BMP signal transduction activator; Item 6. The production method according to any one of items 1 to 5, wherein the culture medium containing a BMP signal transduction activator is a culture medium that contains a BMP signal transduction activator but is free of an FGF signal transduction activator;
Item 7. The production method according to any one of items 1 to 6, wherein the BMP signal transduction activator is Bone Morphogenetic Protein 4;
Item 8. The production method according to any one of items 1 to 7, wherein the cells under culturing is further brought into contact with an FGF signal transduction inhibitor before cells expressing GCM1 mRNA appear in the culture;
Item 9. The production method according to any one of items 1 to 8, wherein at least one selected from the group consisting of a γ aminobutyric acid B receptor activator, a peroxisome proliferator-activated receptor γ activator, a retinoid X receptor activator, and a retinoic acid receptor activator are a γ aminobutyric acid B receptor activator, and at least one selected from the group consisting of a peroxisome proliferator-activated receptor γ activator, a retinoid X receptor activator, and a retinoic acid receptor activator;
Item 10. The production method according to any one of items 1 to 9, wherein at least one selected from the group consisting of a γ aminobutyric acid B receptor activator, a peroxisome proliferator-activated receptor γ activator, a retinoid X receptor activator, and a retinoic acid receptor activator are a γ aminobutyric acid B receptor activator, a peroxisome proliferator-activated receptor γ activator, a retinoid X receptor activator, and a retinoic acid receptor activator;
Item 11. The production method according to any one of items 1 to 10, wherein the human pluripotent stem cells are human pluripotent stem cells that are maintenance cultured after singly dispersed;
Item 12. The production method according to any one of items 1 to 11, wherein the pluripotent stem cells are embryonic stem cells (hereinafter sometimes referred to as ES cells) or induced pluripotent stem cells;
Item 13. Artificial trophoblasts or artificial syncytiotrophoblasts produced by the method according to any one of items 1 to 12;
Item 14. A kit comprising artificial trophoblasts or artificial syncytiotrophoblasts produced by the method according to any one of items 1 to 12;
Item 15. A method for assaying cell layer permeability of a test substance, comprising: bringing the test substance into contact with artificial trophoblasts or artificial syncytiotrophoblasts produced by the method according to any one of items 1 to 12, and assaying permeability of the substance to the trophoblasts or syncytiotrophoblasts;
Item 16. Use of artificial trophoblasts or artificial syncytiotrophoblasts produced by the method according to any one of items 1 to 12 as a reagent for evaluating toxicity or drug efficacy;
Item 17. A method for evaluating toxicity or drug efficacy of a test substance, comprising bringing the test substance into contact with artificial trophoblasts or artificial syncytiotrophoblasts produced by the method according to any one of items 1 to 12, and assaying the influence of the substance on the trophoblasts or syncytiotrophoblasts;
Item 18. A method for analyzing clinical condition of a disease due to a damage of placenta tissue, comprising, by using artificial trophoblasts or artificial syncytiotrophoblasts produced by the method according to any one of items 1 to 12, examining substance transportation or hormone secretion to which the trophoblasts or syncytiotrophoblasts are involved; and
Item 19. Use of artificial trophoblasts or artificial syncytiotrophoblasts produced by the method according to any one of items 1 to 12 as a reagent for analyzing clinical condition of a disease due to a damage of placenta tissue; and so on.

### EFFECT OF THE INVENTION

According to Production method of the present invention, it becomes possible to produce artificial syncytiotrophoblasts derived from human cells, and progenitor cells thereof in vitro with high efficiency. The artificial syncytiotrophoblasts and progenitor cells thereof produced by Production method of the present invention can be utilized for evaluating the toxicity or the drug efficacy of a chemical substance or the like, and for analyzing clinical condition.

### BRIEF DESCRIPTION OF THE DRAWINGS

Fig. 1 is a graph showing the percentage (%) of GFP positive cells when human ES cells in which a nucleotide sequence encoding green fluorescent protein (GFP) is knocked-in downstream the promoter of GCM1 gene are cultured in a differentiation medium.
Fig. 2 is a graph showing the mRNA amount of Syncytin gene in cells obtained by culturing human ES cells in a differentiation medium as a relative expression amount.
Fig. 3 is a graph showing the mRNA amount of Syncytin gene in cells obtained by culturing human ES cells as a relative expression amount.
Fig. 4 shows an image of cells obtained by culturing human ES cells in which a nucleotide sequence encoding green fluorescent protein (GFP) is knocked-in downstream the promoter of GCM1 gene in a differentiation medium, immunostained with an anti-GFP antibody and a nuclear stain.
Fig. 5 is a graph showing the mRNA amounts of various marker genes in cells obtained by culturing human ES cells as relative expression amounts.
Fig. 6 is a graph showing the mRNA amount of GCM1 gene and the mRNA amount of Syncytin gene in cells obtained by culturing human ES cells in a differentiation medium as relative expression amounts.
Fig. 7 is a graph showing the mRNA amount of KRT7 gene, the mRNA amount of GCM1 gene and the mRNA amount of Syncytin gene in cells obtained by culturing human ES cells in a differentiation medium as relative expression amounts over time.
Fig. 8 is a graph showing the percentage (%) of GFP positive cells when human ES cells in which a nucleotide sequence encoding green fluorescent protein (GFP) is knocked-in downstream the promoter of GCM1 gene are cultured in a differentiation medium.
Fig. 9 is a graph showing the mRNA amount of Syncytin gene in cells obtained by culturing human ES cells as a relative expression amount.
Fig. 10 is a graph showing the correlation between the permeation amount of a test substance in cells obtained by culturing human ES cells in a differentiation medium, and the permeation amount (literature value) of the substance determined in the permeation test by the placenta reflux method using term placenta.
Fig. 11 is a graph showing the mRNA amount of Syncytin gene in cells obtained by culturing human induced pluripotent stem cells in a differentiation medium as a relative expression amount.
Fig. 12 is a graph showing the correlation between the permeation amount of a test substance in cells obtained by culturing human induced pluripotent stem cells in a differentiation medium, and the permeation amount (literature value) of the substance determined in the permeation test by the placenta reflux method using term placenta.
Fig. 13 is a graph showing a TEER value in cells obtained by culturing human ES cells in a differentiation medium or in BeWo cells.
Fig. 14 shows an image of cells obtained by culturing human ES cells in a differentiation medium, or BeWo cells, stained with an anti-Mdr1 antibody and a nuclear stain.
Fig. 15 is a graph showing the mRNA amount of BCRP gene in cells obtained by culturing human ES cells in a differentiation medium or in BeWo cells, and the mRNA amount of BCRP gene in placenta total RNA as relative expression amounts.
Fig. 16 is a graph showing the calcein fluorescence intensity in cells obtained by culturing human ES cells or BeWo cells, as a relative amount.

### MODE FOR CARRYING OUT THE INVENTION

Modes for carrying out the present invention is described in detail below.

Examples of prokaryotic cells used in gene engineering techniques in the present invention include cells of prokaryote belonging to Escherichia, Serratia, Bacillus, Brevibacterium, Corynebacterium, Microbacterium, Pseudomonas and so on, such as Eschericia XL1-Blue, Eschericia XL2-Blue, and Eschericia DH1. Such cells are concretely described, for example, in "Molecular Cloning: A Laboratory Manual (3rd edition)" Sambrook, J and Russell, D.W., ed., Appendix 3 (Volume3), Vectors and Bacterial strains. A3.2 (Cold Spring Harbor Laboratory Press, 2001).

The "vector" in the present invention means a vector capable of transferring a desired polynucleotide sequence into an intended cell. Examples of such vector include a vector capable of autonomously replicating in a host cell such as prokaryotic cell, yeast, animal cell, plant cell, insect cell, animal individual and plant individual, or capable of being incorporated into a chromosome, and containing a promoter at a position suitable for polynucleotide transcription, and so on.

Of such vectors, a vector suitable for cloning normally includes a multiple cloning site containing a plurality of restriction enzyme sites. Representative examples of vectors that can be used for cloning of gene are described, for example, in "Molecular Cloning: A Laboratory Manual (3rd edition)" Sambrook, J and Russell, D.W., ed., Appendix 3 (Volume 3), Vectors and Bacterial strains. A3.2 (Cold Spring Harbor Laboratory Press, 2001), and a person skilled in the art can use such vectors appropriately depending on the purpose.

The "vector" in the present invention also includes "expression vector", "reporter vector", and "recombinant vector". The "expression vector" means a nucleic acid sequence in which various regulatory elements in addition to a structural gene and a promoter that regulates the expression of the structural gene are linked in such a manner that they can be operable in the host cell. Examples of the "regulatory element" include nucleotide sequences including a terminator, a selective marker such as a drug resistance gene, and an enhancer.

Examples of the technique for introducing nucleic acid molecules into cells in the present invention include transformation, transduction, and transfection. Concrete examples of such introduction techniques include methods that are described, for example, in Ausubel F. A. et al. ed. (1988) Current Protocols in Molecular Biology, Wiley, New York, NY; "Molecular Cloning: A Laboratory Manual (3rd edition)" Sambrook, J and Russell, D.W., ed., (Cold Spring Harbor Laboratory Press, 2001); or extra issue, Experimental Medicine "Transgene & expression analysis experiment method" YODOSHA CO. , LTD., (1997) . As the technique for confirming that a gene has been introduced into a cell, for example, Northern blot analysis, Western blot analysis or other well-known routine techniques can be recited.

The medium to be used in culturing cells in the present invention can be prepared from a medium used for culturing animal cell as a basal culture medium. Examples of the basal culture medium include BME medium, BGJb medium, CMRL 1066 medium, Glasgow MEM medium, Improved MEM Zinc Option medium, IMDM medium, Medium 199 medium, Eagle MEM medium, αMEM medium, DMEM medium, Ham's medium, F-12 medium, DMEM/F-12 medium, RPMI 1640 medium, Fischer's medium, and mixed media thereof that can be used for culturing animal cells.

The culture medium to be used in culturing cells in the present invention is supplemented with unadjusted or unpurified serum as is necessary. Examples of "serum" include mammalian serums such as bovine serum, calf serum, fetal bovine serum, horse serum, foal serum, fatal horse serum, rabbit serum, leveret serum, fetal rabbit serum, and human serum. The culture medium may further contain fatty acid, lipid, amino acid (for example, nonessential amino acid), vitamin, a growth factor, cytokine, an antioxidant, 2-mercaptoethanol, pyruvic acid, a buffer, inorganic salts and so on.

In the present invention, "culture medium containing substance X" means a culture medium supplemented with exogenous substance X, or a culture medium containing exogenous substance X, and "culture medium free of substance X" means a culture medium not supplemented with exogenous substance X or a culture medium not containing exogenous substance X. Here, "exogenous substance X" means substance X that is exogenous to the cells or tissue to be cultured in the culture medium, and endogenous substance X produced by the cells or tissue is not included in "exogenous substance X".

For example, "culture medium containing a BMP signal transduction activator" means a culture medium supplemented with an exogenous BMP signal transduction activator or a culture medium containing an exogenous BMP signal transduction activator. The "culture medium free of an FGF signal transduction activator" means a culture medium not supplemented with an exogenous FGF signal transduction activator, or a culture medium not containing an exogenous FGF signal transduction activator.

Production method 1 of the present invention is a method for producing artificial trophoblasts derived from human cells, and comprises the steps of: adhesion culturing human pluripotent stem cells in a culture medium containing a BMP signal transduction activator, and bringing the cells under culturing into contact with at least one selected from the group consisting of a γ aminobutyric acid B receptor activator, a peroxisome proliferator-activated receptor γ activator, a retinoid X receptor activator, and a retinoic acid receptor activator during the culturing, thereby obtaining a culture containing trophoblasts differentiated from the human pluripotent stem cells.

Production method 2 of the present invention is a method for producing artificial syncytiotrophoblasts derived from human cells, and comprises the steps of: adhesion culturing human pluripotent stem cells in a culture medium containing a BMP signal transduction activator, and bringing the cells under culturing into contact with at least one selected from the group consisting of a γ aminobutyric acid B receptor activator, a peroxisome proliferator-activated receptor γ activator, a retinoid X receptor activator, and a retinoic acid receptor activator during the culturing, thereby obtaining a culture containing syncytiotrophoblasts differentiated from the human pluripotent stem cells.

In the present invention, the "stem cell" refers to a cell that has an ability to divide and produce a cell that is identical to itself, namely the self-replicating ability, and an ability to differentiate into other type of cell, and is capable of continuously proliferating.

In the present invention, the "pluripotent stem cell" refers to a stem cell that can be cultured in vitro and has an ability to differentiate into tissues derived from three germ layers (ectoderm, mesoderm, endoderm), namely pluripotency. The "pluripotent stem cell" can be established, for example, from a fertilized egg, a clone embryo, a reproductive stem cell, and a stem cell in tissue. More concrete examples of the "pluripotent stem cell" in the present invention include an embryonic stem cell, or a pluripotent stem cell induced from a somatic cell.

The "human pluripotent stem cell" in the present invention means a pluripotent stem cell derived from a human cell. For example, a pluripotent stem cell established from a human fertilized egg, a clone embryo, a reproductive stem cell, or a stem cell in tissue, or a pluripotent stem cell established from a human somatic cell can be recited.

Examples of the "embryonic stem cell" (hereinafter, sometimes referred to as ES cell) in the present invention include a pluripotent stem cell that is a stem cell having a self-replicating ability and pluripotency, and is derived from an early embryo. An embryonic stem cell was first established in 1981, and has also been applied to the generation of knockout mouse since 1989. In 1998, a human embryonic stem cell was established, which is also being utilized for regenerative medicine.

An embryonic stem cell is a cell that was established from an inner cell mass which is a population of cells to differentiate into embryonic tissue such as a fetus in future, and it has been considered heretofore that an embryonic stem cell does not differentiate into extraembryonic tissue such as a placenta. However, in recent years, the possibility that an embryonic stem cell can be differentiated into extraembryonic tissue including a placenta by being cultured in specific conditions. For example, there has been a report that by culturing human ES cells while they are brought into contact with a culture medium supplemented with BMP4 (Nature Biotechnology, 2002, 20, p1261-1264), differentiation into cells of trophectoderm lineage that are to be differentiated into extraembryonic tissue such as placenta occurs.

As the "pluripotent stem cell induced from a somatic cell" in the present invention, a cell to which pluripotency similar to that of an embryonic stem cell is artificially imparted by initializing a somatic cell can be recited, and concrete examples include an induced pluripotent stem cell (hereinafter, sometimes referred to as iPS cell) in which pluripotency is induced by initializing a differentiated cell such as a fibroblast or the like by expression of a gene such as Oct3/4, Sox2, Klf4, or Myc. In 2006, induced pluripotent stem cell was established from mouse fibroblast by Yamanaka et. al. (Cell, 2006, 126(4), p663-676). In 2007, induced pluripotent stem cell having multipotency similarly to embryonic stem cell was established from human fibroblast (Cell, 2007, 131(5), p861-872; Science, 2007, 318(5858), p1917-1920; Nat Biotechnol., 2008, 26(1), p101-106).

Pluripotent stem cells are available from given organizations, or commercially available products can also be purchased. For example, human embryonic stem cells, KhES-1, KhES-2 and KhES-3, are available from Kyoto University's Institute for Frontier Medical Sciences.

Pluripotent stem cells can be maintenance cultured according to a method known per se. For example, human stem cells can be maintained by culturing by using KnockOut™ Serum Replacement (hereinafter, sometimes referred to as KSR) and a basic fibroblast growth factor (hereinafter, sometimes referred to as bFGF).

The pluripotent stem cells used in the present invention may be pluripotent stem cells that are genetically modified. The genetically modified pluripotent stem cells can be prepared, for example, by using a homologous recombination technique. Examples of the gene on the chromosome to be modified include a cell marker gene, a histocompatibility antigen gene, a gene related to a diseasedue to a disorder of placenta tissue and so on. Examples of the genetic modification include the modification of knocking in a nucleotide sequence encoding a reporter protein downstream the promoter region of the intended cell marker gene for the purpose of expressing the reporter gene under the control of the promoter of the cell marker gene, and the modification of introducing mutation into a disease-related gene. Examples of the reporter protein include enzymes such as firefly luciferase (firefly luc), renilla luciferase (renilla luc), β-galactosidase, or chloramphenicol acetyl transferase, and fluorescent proteins such as green fluorescent protein (GFP), blue fluorescent protein (CFP), yellow fluorescent protein (YFP) or red fluorescent protein (dsRed). Examples of genetically modified pluripotent stem cells include a pluripotent stem cell in which a nucleotide sequence encoding a reporter protein such as GFP is knocked in downstream the promoter of GCM1 gene which is one of marker genes of syncytiotrophoblast.

Modification of a target gene on chromosome can be conducted by a method described, for example, in Manipulating the Mouse Embryo, A Laboratory Manual, Second Edition, Cold Spring Harbor Laboratory Press(1994); Gene Targeting, A Practical Approach, IRL Press at Oxford University Press (1993); Bio Manual series 8, gene targeting, Production of mutant mouse by using ES cells, YODOSHA CO., LTD. (1995) and so on.

Concretely, for example, a genomic gene of a target gene to be modified (for example, cell marker gene, histocompatibility antigen gene, disease-related gene and so on) is isolated, and a target vector for homologous recombination of the target gene is prepared by using the isolated genomic gene. The prepared target vector is introduced into stem cells, and cells in which homologous recombination occurs between the target gene and the target vector are selected, whereby stem cells having modified gene on the chromosome can be prepared.

As a method for isolating the genomic gene of the target gene, known methods described in Molecular Cloning, A Laboratory Manual, Second Edition, Cold Spring Harbor Laboratory Press (1989), Current Protocols in Molecular Biology, John Wiley & Sons (1987-1997) and so on can be mentioned. Moreover, the genomic gene of the target gene can be isolated using genomic DNA library screening system (manufactured by Genome Systems), Universal GenomeWalker Kits (manufactured by CLONTECH) and so on.

Preparation of a target vector used for homologous recombination of the target gene, and efficient selection of a homologous recombinant can be conducted according to the methods described in Gene Targeting, A Practical Approach, IRL Press at Oxford University Press (1993) ; Bio Manual series 8, gene targeting, Production of mutant mouse by using ES cells, YODOSHA CO., LTD. (1995) and so on. The target vector may be any of replacement type and insertion type. The selection method may be positive selection, promoter selection, negative selection, polyA selection and so on.

As a method for selecting an intended homologous recombinant from the selected cell lines, Southern hybridization method, PCR method and so on for genomic DNA can be mentioned.

In Production method 1 of the present invention and Production method 2 of the present invention (hereinafter, sometimes collectively referred to as Production method of the present invention), human pluripotent stem cells are adhesion cultured in a culture medium containing a BMP signal transduction activator. The culturing (hereinafter, sometimes referred to as Present differentiation culturing) will be described.

The "adhesion culturing" in the present invention means culturing in the condition that the cells are adhered to a culture vessel material or the like.

The form of the culture vessel material used in Present differentiation culturing is not particularly limited as long as the culture vessel material allows adhesion culturing of cells. Examples of such a culture vessel material include flask, tissue culture flask, dish, petri dish, tissue culture dish, multidish, microplate, microwell plate, micropore, multiplate, multiwell plate, chamber slide, schale, tube, tray, culture bag, roller bottle, cell culture insert, and Organ-on-a-Chip.

Examples of a preferred culture vessel material include a cell-adhesive culture vessel material. Examples of the cell-adhesive culture vessel material include a cell culture vessel material in which the culture surface is artificially treated so as to improve the adhesiveness with cells. Examples of the treatment for improving the adhesiveness with cells include a coating treatment with an extracellular matrix or the like. Examples of the extracellular matrix or the like for use in coating include products that are commercially available as basement membrane components (for example, Matrigel™; manufactured by Beckton Dickinson), and extracellular matrix molecules known as basement membrane components (e.g., typ I collagen, laminin, type IV collagen, heparan sulfate proteoglycan, entactin and so on). Cells can be adhered, for example, to the bottom surface, lateral surface, back side of the bottom surface (for example, back side of a cup of a detachable cell culture insert) or the like of the vessel material, and the adhesion site is not particularly limited as long as the cells are not in floating conditions.

Matrigel™ is a basement membrane preparation derived from Engelbreth Holm Swarn (EHS) mouse sarcoma. Main components of Matrigel™ are type IV collagen, laminin, heparan sulfate proteoglycan, and entactin, and in addition to these, TGF-β, a fibroblast growth factor (FGF), a tissue plasminogen activator, and a growth factor naturally produced by EHS tumor are contained. The "growth factor reduced (GFR) product" of Matrigel™ has a lower growth factor concentration than common Matrigel™. In the present invention, it is preferred to use the GFR product.

In Present differentiation culturing, a culture medium in which a BMP signal transduction activator is added to a culture medium as described above is used. For example, a DMEM/F-12 medium supplemented with a BMP signal transduction activator in addition to 10% fetal bovine serum (hereinafter, sometimes referred to as FBS), 2 mM L-glutamine, 100 U/ml penicillin and 100 µg/ml streptomycin can be recited.

The "BMP signal transduction activator" refers to a substance capable of enhancing signal transduction mediated by BMP. Examples of the BMP signal transduction activator include BMP such as BMP2, BMP4 or BMP7, GDF protein such as growth differentiation factor 7 (GDF7), anti-BMP receptor antibody, or BMP partial peptide. BMP2, BMP4 and BMP7 are available, for example, from R&D Systems, and GDF7 protein is available, for example, from Wako Pure Chemical Industries, Ltd..

Concentration of the BMP signal transduction activator can be such a concentration that can induce differentiation of human pluripotent stem cells to trophoblasts. For example, BMP4 is added to the culture medium so that the concentration in the culture medium is about 1 ng/ml to about 1000 ng/ml, preferably about 10 ng/ml to about 300 ng/ml, more preferably about 100 ng/ml.

The "culture medium containing a BMP signal transduction activator" used in Present differentiation culturing is preferably a culture medium that contains a BMP signal transduction activator but is free of an FGF signal transduction activator.

The "FGF signal transduction activator" refers to a substance capable of enhancing signal transduction mediated by FGF. Examples of the FGF signal transduction activator include proteins belonging to FGF family (for example, a basic fibroblast growth factor) and FGF-like substances (for example, SUN 11602).

Human pluripotent stem cells are adhesion cultured in the "culture medium containing a BMP signal transduction activator" as described above by using a culture vessel material as described above. The cell concentration at the time of starting Present differentiation culturing is, for example, about 5 × 10² cells to about 2 × 10⁴ cells, preferably about 7.5 × 10² cells to about 6 × 10³ cells, more preferably about 1 × 10³ cells to about 4 × 10³ cells per 1 well of a 96-well culture plate.

As the human pluripotent stem cells used in Present differentiation culturing, cells that are maintenance cultured after singly dispersed are preferred.

The "singly dispersed cells" mean the cells that are not in a mass of cells (colony) but are dispersed to the conditions of single cells. As a method of singly dispersing cells, a method of treating a mass of cells with a cell dispersing reagent, or the like can be recited. For example, a method of treating with enzyme such as trypsin-EDTA, or a trypsin alternative such as TrypLE Express (Life Technologies), or a method of pipetting after treating with a cell dispersing reagent not containing enzyme can be recited. A method that inflicts less damage on cells is preferred.

After maintenance culturing human pluripotent stem cells that are singly dispersed for a certain term while they are undifferentiated, the cells are subjected to Present differentiation culturing. The cell concentration at the start of maintenance culturing of the singly dispersed human pluripotent stem cells is, for example, about 5 × 10² cells to about 1 × 10⁴ cells, preferably about 7.5 × 10² cells to about 3 × 10³ cells, more preferably about 1 × 10³ cells to about 2 × 10³ cells per 1 well of a 96-well culture plate. As a method of maintenance culturing, a method of adhesion culturing human pluripotent stem cells in a culture medium that is ordinarily used for culturing pluripotent stem cells in undifferentiated conditions can be recited. For example, after human pluripotent stem cells that have singly dispersed and seeded adhere to the culture vessel material and start proliferating and before a mass of cells consisting of about 100 cells is formed, the maintenance culturing is terminated and Present differentiation culturing is started. Present differentiation culturing is started before a mass of cells consisting of preferably about 50 cells, more preferably about 20 cells is formed. While the concrete number of days of maintenance culturing differs depending on the type and condition of the cells to be cultured, the components of the culture medium, and the culture conditions and so on, for example, about one day to about three days can be recited.

For example, after singly dispersing a colony of human pluripotent stem cells by using a cell dispersing reagent or the like, the cells are suspended in a culture medium and seeded in a 96-well culture plate for adhesion culturing so that about 1.5 × 10³ cells are contained per 1 well, and maintenance cultured at 37°C, 2% CO₂. As the culture medium at this time, for example, a culture medium in which 10 ng/ml bFGF and 20 µM Y-27632 are added to the supernatant of the overnight culture of mouse fibroblasts (hereinafter sometimes referred to as MEF) in a DMEM/F-12 medium containing 20% KSR or the like is used. For example, after two days from the start of maintenance culturing, the culture medium is replaced by a culture medium containing a BMP signal transduction activator as described above, and Present differentiation culturing is started.

In Production method of the present invention, during Present differentiation culturing, the cells under culturing are brought into contact with at least one selected from the group consisting of a γ aminobutyric acid B receptor activator, a peroxisome proliferator-activated receptor γ activator, a retinoid X receptor activator, and a retinoic acid receptor activator.

The contact between the cells and the substance is conducted in a part of the period or over the entire period of Present differentiation culturing. Preferably, the contact is started before cells expressing GCM1 mRNA appear in the culture of Present differentiation culturing. GCM1 mRNA refers to mRNA encoding glial cells missing 1 which is a DNA binding protein. While the contact may be started before cells expressing GCM1 mRNA appear in the culture of Present differentiation culturing, and terminated before cells expressing GCM1 mRNA appear, preferably the contact is conducted continuously from before cells expressing GCM1 mRNA appear to after appearance of cells expressing GCM1 mRNA, and more preferably the contact is conducted over the entire period of Present differentiation culturing.

Whether cells expressing GCM1 mRNA appear in the culture of Present differentiation culturing can be observed by determining whether cells existing in the culture system contain GCM1 mRNA by conventional biochemical techniques. Concretely, whether GCM1 mRNA is expressed can be examined by extracting total RNA from the cells existing in the culture system, conducting reverse transcription reaction, and conducting real-time PCR by using a primer capable of detecting GCM1 mRNA. As will be described in later-described Example 1, when human pluripotent stem cells in which a nucleotide sequence encoding GFP is knocked in GCM1 gene locus are used, expression of GCM1 mRNA can be observed by detecting fluorescence emitted by GFP. For example, if GCM1 mRNA is detected from the cells existing in the culture system by conventional biochemical techniques, it is determined that the cells expressing GCM1 mRNA appear in the culture of Present differentiation culturing. In the case where the timing at which cells expressing GCM1 mRNA appear in the culture of Present differentiation culturing is known in advance, the contact between the cells and the substance can be conducted at a predetermined timing without conducting the examining operation as described above.

The period "before cells expressing GCM1 mRNA appear in the culture" of Present differentiation culturing differs depending on the type and condition of the cells to be cultured, the components of the culture medium, and the culturing conditions and so on, for example, within two days to three days after starting of Present differentiation culturing can be recited. For example, the contact between the cells and the substance is started simultaneously with the start of Present differentiation culturing or within two days to three days from the start of Present differentiation culturing. The contact may be terminated after about one day, however, the contact is preferably continued for two days or more.

In Production method of the present invention, the cells under culturing may further be brought into contact with an FGF signal transduction inhibitor before cells expressing GCM1 mRNA appear in the culture. For example, the contact is conducted until day 3 from the start of the Present differentiation culturing. The contact between the cells and the FGF signal transduction inhibitor may be conducted at the same timing with the contact with the cells and at least one selected from the group consisting of a γ aminobutyric acid B receptor activator, a peroxisome proliferator-activated receptor γ activator, a retinoid X receptor activator, and a retinoic acid receptor activator.

For bringing the cells into contact with the substance, for example, the cells are cultured in a culture medium containing the substance. Concretely, the substance is added to the culture medium in which the cells are cultured, or the culture medium in which the cells are cultured is replaced by a culture medium containing the substance.

The "γ aminobutyric acid (hereinafter, sometimes referred to as GABA) B receptor activator" in the present invention refers to a substance capable of activating a GABA B receptor. Examples of the GABA B receptor activator include a GABA B receptor agonist and a GABAB receptor positive allosteric modulator (hereinafter, sometimes referred to as PAM) .

Example of the GABA B receptor agonist include GABA, a GABA analog such as γ-Amino-β-hydroxybutyric acid, Baclofen, SKF 97541, or Acamprosate. The GABA B receptor agonist is added to the culture medium so that the concentration in the culture medium is about 10 µM to about 1 mM, preferably about 300 µM, for example, in the case of Baclofen.

Examples of the GABAB receptor PAM include CGP 7930, rac BHFF, CGP 13501, or GS 39783. The GABAB receptor PAM is added to the culture medium so that the concentration is about 10 nM to about 100 µM, preferably about 0.1 µM to about 10 µM, for example, in the case of CGP 7930.

The "peroxisome proliferator-activated receptor γ (hereinafter, sometimes referred to as PPARγ) activator" refers to a substance capable of activating PPARγ. Examples of the PPARγ activator include a PPARγ agonist. Examples of the PPARγ agonist include S26948, GW 1929, Ciglitazone, LG 100754, nTZDpa, Pioglitazone, 15-deoxy-Δ-12,14-Prostaglandin J2, Rosiglitazone, Telmisartan, or Troglitazone.

The PPARγ activator is added to the culture medium so that the concentration in the culture medium is about 10 nM to about 10 µM, preferably about 100 nM to about 1 µM, for example, in the case of S26948.

The "retinoid X receptor (hereinafter, sometimes referred to as RXR) activator" in the present invention refers to a substance capable of activating RXR. Examples of the RXR activator include an RXR agonist. Examples of the RXR agonist t include SR 11237, Fluorobexarotene, Docosahexaenoic acid, Isotretinoin, or CD 3254.

The RXR activator is added to the culture medium so that the concentration in the culture medium is about 1 nM to about 1 µM, preferably about 10 nM to about 100 nM, for example, in the case of SR 11237.

The "retinoic acid receptor (hereinafter, sometimes referred to as RAR) activator" in the present invention refers to a substance capable of activating RAR. Examples of the RAR activator include an RAR agonist. Examples of the RAR agonist include retinoic acid, BMS 753, AM 580, Ch 55, AC 261066, AC 55649, Adapalene, AM 80, BMS 961, CD 1530, CD 2314, CD 437, Isotretinoin, Tazarotene or TTNPB.

The RAR activator is added to the culture medium so that the concentration in the culture medium is about 0.1 nM to about 100 nM, preferably about 1 nM to about 10 nM, for example, in the case of retinoic acid.

The FGF signal transduction inhibitor refers to a substance capable of inhibiting signal transduction mediated by FGF. Examples of the FGF signal transduction inhibitor include an FGF receptor inhibitor. The FGF receptor inhibitor refers to a substance capable of inhibiting the function of an FGF receptor. Examples of the FGF receptor inhibitor include PD 173074, SU-5402, AP 24534, FIIN 1 hydrochloride, PD 161570, PD 166285 dihydrochloride, R 1530, or SU 6668.

The FGF signal transduction inhibitor is added to the culture medium so that the concentration is about 1 nM to about 10 µM, preferably about 0.1 µM to about 3 µM, for example, in the case of PD173074.

The "at least one selected from the group consisting of a GABA B receptor activator, a PPARγ activator, an RXR activator, and an RAR activator" are, for example, at least one selected from the group consisting of a PPARγ activator, an RXR activator, and an RAR activator, and a GABA B receptor activator, and preferably, a GABA B receptor activator, a PPARγ activator, an RXR activator, and an RAR activator. The at least one substance to be brought into contact with the cells can include two or more different substances which are a GABA B receptor activator, a PPARγ activator, an RXR activator, or an RAR activator, respectively.

The culturing conditions such as culturing temperature, and CO₂ concentration in the maintenance culturing and Present differentiation culturing can be appropriately determined individually. While the culturing temperature is not particularly limited, it is, for example, about 30°C to about 40°C, preferably about 37°C. The CO₂ concentration is, for example, about 1% to about 10%, preferably about 2% to about 5%. The O₂ concentration is, for example, about 20% to about 70%, preferably about 20% to about 60%, more preferably about 20%.

In Production method of the present invention, human pluripotent stem cells are adhesion cultured in a culture medium containing a BMP signal transduction activator, and the cells under culturing are brought into contact with at least one selected from the group consisting of a γ aminobutyric acid B receptor activator, a peroxisome proliferator-activated receptor γ activator, a retinoid X receptor activator, and a retinoic acid receptor activator during the culturing. By conducting the culturing as described above, trophoblasts differentiated from the human pluripotent stem cells appear in the culture, and thereafter, further differentiated syncytiotrophoblasts appear.

In Production method of the present invention, Present differentiation culturing is continued while the culture medium is appropriately replaced until appearance of intended cells, namely trophoblasts or syncytiotrophoblasts is observed in the culture, and thus a culture containing the intended cells is obtained. In the case where the timing at which the intended cells appear in the culture is known in advance, the culture containing desired cells can be obtained at a predetermined timing without conducting the confirmation operation as will be described later.

The "trophoblasts differentiated from human pluripotent stem cells" produced by Production method 1 of the present invention are trophoblasts before forming syncytiotrophoblasts by fusion, namely progenitor cells of syncytiotrophoblasts, and may include cytotrophoblasts. The "trophoblasts differentiated from human pluripotent stem cells" can be observed as cells expressing mRNA encoding Cytokeratin7 (hereinafter, sometimes referred to as KRT7) but not expressing mRNA encoding Syncytin. Whether cells expressing express KRT7 mRNA but not expressing Syncytin mRNA appear in the culture can be examined by determining whether cells existing in the culture system contain the two kinds of mRNA by conventional biochemical techniques. Concretely, whether KRT7 mRNA and Syncytin mRNA are expressed can be examined by extracting total RNA from the cells existing in the culture system, conducting reverse transcription reaction, and conducting real-time PCR by using primers capable of detecting KRT7 mRNA and Syncytin mRNA, respectively. For example, if KRT7 mRNA is detected, and Syncytin mRNA is not detected from the cells existing in the culture system by conventional biochemical techniques, it is determined that the cells expressing KRT7 mRNA but not expressing Syncytin mRNA appear in the culture of Present differentiation culturing.

As the "culture containing trophoblasts differentiated from human pluripotent stem cells" obtained by Production method 1 of the present invention, a culture containing trophoblasts differentiated from human pluripotent stem cells but not containing syncytiotrophoblasts differentiated from human pluripotent stem cells can be recited. While the culturing period required for obtaining such a culture differs depending on the culturing conditions, the type of culture medium, the type and the condition of cells and the like, it is, for example, about one day to about two days from the start of Present differentiation culturing.

The cells contained in the "culture containing trophoblasts differentiated from human pluripotent stem cells" obtained by Production method 1 of the present invention can be utilized for evaluation of toxicity or drug efficacy, analysis of clinical condition, or therapeutic drug screening or the like. It is also possible to collect cells from the culture, plate the collected cells on a culture vessel material suited for the purpose, further culture as necessary, and then use the cells for evaluation of toxicity or drug efficacy, analysis of clinical condition, or therapeutic drug screening, and so on. The cells collected from the culture can be components of a kit as will be described later. Trophoblasts with higher purity can be obtained by screening the cells collected from the culture by an appropriate cell separation technique.

The "syncytiotrophoblasts differentiated from human pluripotent stem cells" produced by Production method 2 of the present invention show the morphology in which the trophoblasts are fused one another, which can be examined by microscopic observation or the like.

Differentiation to syncytiotrophoblasts can also be examined by determining expression of a marker gene by conventional biochemical techniques. Examples of the method for determining expression of a marker gene include a method of extracting total RAN from a culture containing cells, conducting a reverse transcription reaction, and conducting real-time PCR by using the obtained DNA as a template, to thereby examine whether the marker gene is expressed or examine the degree of expression, and a method of immunostaining cells using an antibody against the protein encoded by the marker gene, to thereby examine whether the marker gene is expressed or examine the degree of expression.

Examples of the marker gene for syncytiotrophoblasts include GCM1 gene, Syncytin1 gene, CGA gene, CGB gene, CSH1 gene, HOPX gene, and TFAP2A gene.

While the culturing period required for obtaining a culture containing "syncytiotrophoblasts differentiated from human pluripotent stem cells" differs depending on the culturing conditions, the type of culture medium, the type and the condition of cells and the like, it is normally about three days to about twelve days, preferably about four days to about six days from the start of Present differentiation culturing.

The cells contained in the "culture containing syncytiotrophoblasts differentiated from human pluripotent stem cells" obtained by Production method 2 of the present invention can be utilized for evaluation of toxicity or drug efficacy, analysis of clinical condition, or therapeutic drug screening, and so on. It is also possible to collect cells from the culture, plate the collected cells on a culture vessel material suited for the purpose, further culture as necessary, and then use the cells for evaluation of toxicity or drug efficacy, analysis of clinical condition, or therapeutic drug screening or the like. The cells collected from the culture can be components of a kit as will be described later. Syncytiotrophoblasts with higher purity can be obtained by screening the cells collected from the culture by an appropriate cell separation technique.

The present invention also include use of artificial trophoblasts or artificial syncytiotrophoblasts produced by Production method of the present invention as a reagent for evaluating toxicity or drug efficacy, use of artificial trophoblasts or artificial syncytiotrophoblasts produced by Production method of the present invention as a reagent for analyzing clinical condition of a disease due to a damage of placenta tissue, and a kit containing artificial trophoblasts or artificial syncytiotrophoblasts produced by Production method of the present invention.

The artificial trophoblasts or artificial syncytiotrophoblasts produced by Production method of the present invention can be utilized for an in vitro human placenta permeability test method, evaluation of toxicity or drug efficacy by the test method, evaluation of toxicity or drug efficacy on human placenta cells, analysis of clinical condition of a disease due to a damage of placenta tissue, therapeutic drug screening for the disease and so on. For example, by bringing a test substance into contact with the artificial trophoblasts or artificial syncytiotrophoblasts produced by Production method of the present invention, and assaying the permeability of the substance to the trophoblasts or syncytiotrophoblasts, or the influence of the substance on the trophoblasts or syncytiotrophoblasts, the toxicity or the drug efficacy of the substance is evaluated. By testing, for example, the substance permeability, cell proliferation or gene expression and so on regarding the trophoblasts or syncytiotrophoblasts, and examining in vivo substance transportation or hormone secretion and the like to which the trophoblasts or syncytiotrophoblasts are involved by using the artificial trophoblasts or artificial syncytiotrophoblasts produced by Production method of the present invention, clinical condition of a disease due to a damage of placenta tissue is conducted.

Examples of the disease due to a damage of placenta tissue include pregnancy-induced hypertension syndrome (PIH) including preeclampsia, gestational hypertension, superimposed preeclampsia and eclampsia, intrauterine growth retardation (IUGR), choriocarcinoma, hydatidiform mole, and hydatid pregnancy.

The kit containing the artificial trophoblasts or artificial syncytiotrophoblasts produced by Production method of the present invention can be utilized for the placenta permeability test method, evaluation of toxicity or drug efficacy, analysis of clinical condition of a disease due to a damage of placenta tissue, therapeutic drug screening for the disease and so on as described above. The kit can be attached with other vessel material, reagent or the like as necessary besides the artificial trophoblasts or artificial syncytiotrophoblasts produced by Production method of the present invention. Examples of other vessel material, reagent or the like include culture vessel material capable of evaluating the permeability of a compound (for example, Transwell-Clear of Corning Cat.3470), culture vessel materials in which wells for culturing the artificial trophoblasts or artificial syncytiotrophoblasts produced by Production method of the present invention, and wells for culturing cells other than the above trophoblasts or syncytiotrophoblasts are connected (for example, Organ-on-a-Chip), or culture media used for restoring or culturing frozen cells.

The present invention is explained in more detail in the following by referring to Examples, which are not to be construed as limitative.

### EXAMPLES

### Example 1: Establishment of GCM1 knock-in human ES cells

A human ES cell line in which a nucleotide sequence encoding GFP is knocked in in a GCM1 gene locus which is one marker gene of syncytiotrophoblast was prepared in the following manner.

A mRNA encoding Zinc Finger Nuclease (ZFN) was purchased from Sigma-Aldrich. Zinc Finger Nuclease (ZFN) targets the nucleotide sequence (the nucleotide sequence of SEQ ID NO: 1; TGGCCTGACCTTATCatggaaCCTGACGACTTTGAT) spanning from 15 bases upstream the translation initiation point to 20 bases downstream the translation initiation point in GCM1 gene on genomic DNA of human ES cell line KhES-1 (available from Kyoto University) . A knock-in vector containing AcGFP gene (TAKARA BIO) and a neomycin resistance gene was prepared. To a singly dispersed human ES cell line KhES-1, the aforementioned mRNA encoding ZFN, and the prepared knock-in vector were co-introduced by electroporation, and the cells after the introduction treatment were seeded on neomycin resistant mouse fibroblasts (Oriental Yeast Co., Ltd.) treated with mitomycin C (Sigma-Aldrich) . From the next day of seeding, G418 (Nacalai Tesque) was added to the culture medium to conduct drug selection. Colonies of the obtained resistant clones were picked up, and kept cultured, and cells in which the nucleotide sequence encoding AcGFP is knocked-in directly after the initiation codon in the GCM1 coding region on only either one of allelic loci were selected by the PCR method. The selected cells are hereinafter referred to as GCM1-GFP knock-in human ES cells.

### Example 2: Production of syncytiotrophoblasts differentiated from human ES cells

### (1) Preparation of GCM1-GFP knock-in human ES cells

GCM1-GFP knock-in human ES cells prepared in Example 1 were seeded on mouse fibroblasts (REPROCELL) treated with mitomycin C and maintenance cultured at 37°C, 2% CO₂ in accordance with the method described in "Ueno, M. et al. PNAS 2006, 103(25), 9554-9559", "Watanabe, K. et al. Nat Biotech 2007, 25, 681-686". As a culture medium at this time, a culture medium in which 20% KnockOut™ Serum Replacement (KSR, Invitrogen), 0.1 mM nonessential amino acids (NEAA, Invitrogen), 2 mM L-glutamine (Sigma-Aldrich), and 0.1 mM 2-mercaptoethanol (Wako) are added to a DMEM/F12 medium (Sigma-Aldrich)(hereinafter, referred to as hES medium) was used with addition of 10 ng/ml bFGF (Wako).

The maintenance cultured GCM1-GFP knock-in human ES cells were washed twice with a phosphate-buffered saline (PBS, Invitrogen) while they were adhered to the cell culture dish, and then PBS supplemented with 0.25% trypsin (Invitrogen), 1 mg/ml collagenaseIV(Invitrogen), 20% KSR, and 1 mM CaCl₂ (Nacalai Tesque) was added to the dish, and the cells were incubated for 5 minutes at 37°C, 2% CO₂. After adding a hES medium to the dish, cells were peeled off by pipetting and the culture medium containing the cells was collected, and centrifuged (1000 rpm, 3 minutes). After removing the supernatant from the centrifuged culture, a hES medium supplemented with 20µM Y-27632 (Wako) was added to the deposit to suspend the cell mass, and then the obtained cell mass suspension was seeded on a cell culture dish (BD Falcon) coated with 0.1% gelatin (Sigma-Aldrich), and incubated at 37°C, 2% CO₂ for 1.5 hours to 2 hours. By collecting the GCM1-GFP knock-in human ES cell mass that does not adhere to the dish together with the culture medium, a suspension of GCM1-GFP knock-in human ES cell mass not containing MEF was obtained (hereinafter, this operation is sometimes referred to as a MEF removing operation).

### (2) Induction of differentiation of GCM1-GFP knock-in human ES cells to syncytiotrophoblasts

The GCM1-GFP knock-in human ES cell mass prepared in the manner as described above was dispersed into single cells by using TrypLE Express (Life Technologies), and the obtained cells were suspended in a culture medium of 150 µl so that 1.5 × 10³ cells were contained per one well of a 96-well culture plate, and seeded on a 96-well culture plate (Edge plate, Nunc) coated with 20-fold diluted Matrigel (Growth Factor Reduced, BD Biosciences), and maintenance cultured at 37°C, 2% CO₂. As a culture medium at this time, a culture medium prepared by adding 10 ng/ml bFGF and 20 µM Y-27632 to the supernatant of the overnight culture of MEF in a hES medium (hereinafter, sometimes referred to as MEF-CM) was used. After two days from the start of the maintenance culturing, the culture medium was replaced by a DMEM/F-12 medium supplemented with 10% FBS (CORNING), 2 mM L-glutamine, 100 U/ml penicillin, 100 µg/ml streptomycin (Penicillin-Streptomycin Mixed Solution, Nacalai Tesque), and 3 ng/ml BMP4 (R&D) (hereinafter, referred to as Basal differentiation medium A), or a differentiation medium in which either 0.1% DMSO (Sigma-Aldrich), 1 µM S26948 (PPAPγ agonist, Tocris Bioscience), 1 µM GW 1929 (PPAPγ agonist, Tocris Bioscience), 1 µM CGP 7930 (GABABR positive allosteric modulator, Tocris Bioscience), 1 µM SR 11237 (RXR agonist, Tocris Bioscience), or 1nM retinoic acid (RAR agonist, Sigma-Aldrich) is added to Basal differentiation medium A, and cells were cultured at 37°C, 5% CO₂. After three days from the culture medium replacement, the culture medium was replaced by the differentiation medium having the same composition. Composition of each differentiation medium is shown in Table 1. S26948, GW 1929, CGP 7930 and SR 11237 were dissolved in DMSO so that they were respectively 1 mM, and retinoic acid was dissolved in DMSO so that it was 10 mM, and each DMSO solution was added to Basal differentiation medium A.

**[Table 1]**

| | Differentiation medium composition | Note: |
|---|---|---|
| Control A | Basal differentiation medium A + 0.1% DMSO | Control |
| 1-1 | Basal differentiation medium A + 1 µM S26948 | Production example |
| 1-2 | Basal differentiation medium A + 1 µM GW 1929 | Production example |
| 1-3 | Basal differentiation medium A + 1 µM CGP 7930 | Production example |
| 1-4 | Basal differentiation medium A + 1 µM SR 11237 | Production example |
| Control B | Basal differentiation medium A | Control |
| 1-5 | Basal differentiation medium A + 1 nM retinoic acid | Production example |

| | | |
|---|---|---|
| Basal differentiation medium A: DMEM/F-12 medium supplemented with 10% FBS, 2 mM L-glutamine, 100 U/ml penicillin, 100 µg/ml streptomycin and 3 ng/ml BMP4 | | |

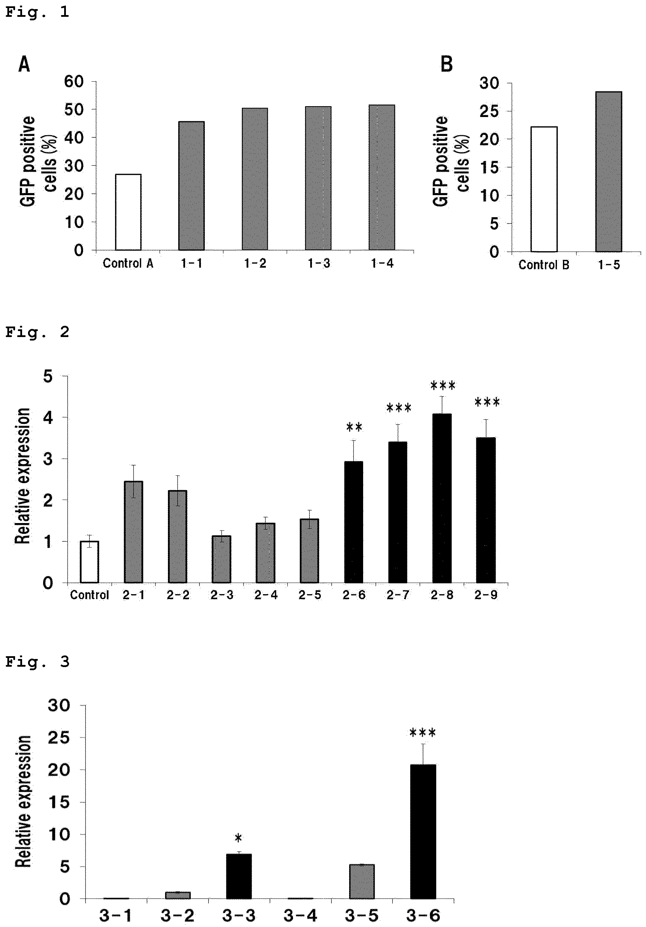

On day 6 of the culturing using the differentiation medium, cells were washed twice with PBS (Invitrogen), and fixed with 4% paraformaldehyde (Wako) for 15 minutes. After subjecting the cells to a membrane permeabilizing treatment for 5 minutes by using PBS supplemented with 0.3% Triton X-100 (Wako), the cells were washed three times with PBS. Then after blocking the cells for 1 hour to 2 hours with 3% Bovine Serum Albumin (BSA, Sigma-Aldrich), the cells were immunostained by using 5 µg/ml chicken anti-GFP antibody (Abcam), 2 µg/ml goat anti-chicken IgG antibody Alexa 488 (Life technologies) and 1µg/ml Hoechst 33342 (DOJINDO). For the obtained stained sample, the percentage of the GFP positive cells indicating expression of GCM1 gene was quantified by using a fully automatic image analyzer Arrayscan (Thermo Scientific). The result is shown in Fig. 1. In comparison with the control to which only DMSO that is a solvent is added (Control A), addition of S26948 (1-1) or GW 1929 (1-2) that is a PPARγ agonist, addition of CGP 7930 (1-3) that is a GABABR positive allosteric modulator, or addition of SR 11237 (1-4) that is an RXR agonist resulted in about 20% of increase in the percentage of the GFP positive cells. Addition of retinoic acid (1-5) that is an RAR agonist resulted in about 6% of increase in the percentage of the GFP positive cells, compared with the control to which retinoic acid is not added (Control B).

### Example 3: Production of syncytiotrophoblasts differentiated from human ES cells

According to the method described in (1) in Example 2, KhES-1 cells were maintenance cultured, and then subjected to the MEF removing operation. The mass of KhES-1 cells having been subjected to the MEF removing operation was dispersed into single cells by using TrypLE Express (Life Technologies), and the obtained cells were suspended in a culture medium of 150 µl so that 1.5 × 10³ cells were contained per one well of a 96-well culture plate, and seeded on a 96-well culture plate (Edge plate, Nunc) coated with 20-fold diluted Matrigel (Growth Factor Reduced, BD Biosciences), and maintenance cultured at 37°C, 2% CO₂. As a culture medium at this time, a culture medium prepared by adding 10 ng/ml bFGF and 20 µM Y-27632 to MEF-CM was used. After two days from the start of the maintenance culturing, the culture medium was replaced by either one of a DMEM/F12 medium supplemented with 10% FBS, 2 mM L-glutamine, 100 U/ml penicillin, 100 µg/ml streptomycin, 100 ng/ml BMP4, and 300 nM PD 173074 (FGF signal transduction inhibitor, Sigma-Aldrich) (hereinafter, referred to as Basal differentiation medium B) ; a differentiation medium prepared by adding either one of 0.1 µM S26948 (PPAPγ agonist), 10 µM GW 1929 (PPAPγ agonist), 0.1 µM CGP 7930 (GABABR positive allosteric modulator), 10 nM SR 11237 (RXR agonist) or 1 nM retinoic acid (RAR agonist) to Basal differentiation medium B; a differentiation medium prepared by adding 0.1 µM S26948, 0.1 µM CGP 7930 and 10 nM SR 11237 to Basal differentiation medium B; a differentiation medium prepared by adding 0.1 µM S26948, 0.1 µM CGP 7930 and 1 nM retinoic acid to Basal differentiation medium B; a differentiation medium prepared by adding 0.1 µM S26948, 0.1 µM CGP 7930, 10 nM SR 11237 and 1 nM retinoic acid to Basal differentiation medium B; or a differentiation medium prepared by adding 10 µM GW 1929, 0.1 µM CGP 7930, 10 nM SR 11237 and 1 nM retinoic acid to Basal differentiation medium B, and the cells were cultured at 37°C, 5% CO₂. After three days from the culture medium replacement, the culture medium was replaced by the differentiation medium having the same composition except that PD 173074 is not contained. Composition of each differentiation medium is shown in Table 2. S26948, GW 1929, CGP 7930 and SR 11237 were dissolved in DMSO so that they were respectively 100 mM, and retinoic acid was dissolved in DMSO so that it was 10 mM, and each DMSO solution was added to Basal differentiation medium B.

**[Table 2]**

| | Differentiation medium composition | Note: |
|---|---|---|
| Control | Basal differentiation medium B | Control |
| 2-1 | Basal differentiation medium B + 0.1 µM S26948 | Production example |
| 2-2 | Basal differentiation medium B + 10 µM GW 1929 | Production example |
| 2-3 | Basal differentiation medium B + 0.1 µM CGP 7930 | Production example |
| 2-4 | Basal differentiation medium B + 10 nM SR 11237 | Production example |
| 2-5 | Basal differentiation medium B + 1 nM retinoic acid | Production example |
| 2-6 | Basal differentiation medium B + 0.1 µM S26948 + 0.1 µM CGP 7930 + 10 nM SR 11237 | Production example |
| 2-7 | Basal differentiation medium B + 0.1 µM S26948 + 0.1 µM CGP 7930+1 nM retinoic acid | Production example |
| 2-8 | Basal differentiation medium B + 0.1 µM S26948 + 0.1 µM CGP 7930 + 10 nM SR 11237 + 1 nM retinoic acid | Production example |
| 2-9 | Basal differentiation medium B + 10 µM GW 1929 + 0.1 µM CGP 7930 + 10 nM SR 11237 + 1 nM retinoic acid | Production example |

| | | |
|---|---|---|
| Basal differentiation medium B: DMEM/F-12 medium supplemented with 10% FBS, 2 mM L-glutamine, 100 U/ml penicillin, 100 µg/ml streptomycin, 100 ng/ml BMP4, and 300nM PD 173074 | | |

On day 5 of the culturing of the culture using the differentiation medium, total RNA was extracted from cells by using RNeasy Micro Kit (QIAGEN). The obtained total RNA was reverse transcribed by using SuperScript III(Invitrogen), and by real-time PCR using the obtained DNA as a template, TaqMan probe (Applied biosystems) and TaqMan Fast advanced master mix (Applied biosystems), the expression amount of Syncytin gene which is a marker gene of syncytiotrophoblast was examined. For the cells obtained in each culturing, the mRNA amount of Syncytin gene and the mRNA amount of GAPDH gene which is a house keeping gene were determined by real-time PCR. The value obtained by dividing the mRNA amount of Syncytin gene by the mRNA amount of GAPDH gene is defined as a correction value of mRNA amount of Syncytin gene, and taking the Syncytin gene mRNA amount correction value in cells cultured in Basal differentiation medium B (Control) as 1, a Syncytin gene mRNA amount correction value in cells cultured in each of other culture media was shown as a relative expression amount. The operation from the cell culturing to measurement of mRNA amount was repeated three times. The result is shown in Fig. 2. In Fig. 2, a standard error of relative expression amount is indicated by an error bar. The relative expression amount having a statistically significant difference with respect to the relative expression amount of Control is marked with asterisks (** means a P value of 0.001 or more and less than 0.01, and *** means a P value of less than 0.001). In comparison with the cells cultured in Basal differentiation medium B (Control), the relative expression amount of Syncytin gene increased in cells that were cultured in a differentiation medium supplemented with either of S26948 (2-1) or GW 1929 (2-2) that is a PPAPγ agonist, CGP 7930 (2-3) that is a GABABR positive allosteric modulator, SR 11237 (2-4) that is an RXR agonist or retinoic acid (2-5) that is an RAR agonist. In the cells cultured in differentiation media supplemented with S26948 or GW 1929 that is a PPARy agonist, and two or more selected from the group consisting of CGP 7930 that is a GABABR positive allosteric modulator, SR 11237 that is an RXR agonist and retinoic acid that is an RAR agonist (2-6 and 2-7), the relative expression amount of Syncytin gene further increased. In the cells cultured in differentiation media supplemented with S26948 or GW 1929 that is a PPARy agonist, and CGP 7930 that is a GABABR positive allosteric modulator, SR 11237 that is an RXR agonist and retinoic acid that is an RAR agonist (2-8 and 2-9), the relative expression amount of Syncytin gene still further increased.

### Example 4: Production of syncytiotrophoblasts differentiated from human ES cells

According to the method described in (1) in Example 2, KhES-1 cells were maintenance cultured, and then subjected to the MEF removing operation.

The mass of KhES-1 cells having been subjected to the MEF removing operation was dispersed into single cells by using TrypLE Express (Life Technologies), and the obtained cells were suspended in 150 µl of a culture medium so that 1.5 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂.

On the other hand, the mass of KhES-1 cells having been subjected to the MEF removing operation was made into cell masses each consisting of several tens of cells by conducting pipetting 10 to 20 times with the use of a 1000 µl filter chip (NPPON Genetics), and the obtained cell mass was suspended in 150 µl of a culture medium so that about 1.5 × 10³ cells to about 4 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂.

In every cell seeding condition as described above, for the maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal differentiation medium B (DMEM/F12 medium supplemented with 10% FBS, 2 mM L-glutamine, 100 U/ml penicillin, 100 µg/ml streptomycin, 100 ng/ml BMP4, and 300 nM PD 173074) (this point of time is day 0), and the cells were cultured at 37°C, 5% CO₂. After three days from the culture medium replacement, the culture medium was replaced by the differentiation medium having the same composition except that PD 173074 is not contained, and the cells were further cultured for two days.

As Comparative example, each of the KhES-1 cells seeded in the above two kinds of conditions was maintenance cultured in the same manner as described above, and after two days from the start of the maintenance culturing, the culture medium was replaced by a differentiation medium prepared by adding 10 ng/ml BMP4, 1 µM A83-01 (activin signal transduction inhibitor, Wako) and 0.1 µM PD 173074 (FGF signal transduction inhibitor) to MEF-CM (described in Non-patent document 2) (this point of time is day 0), then the cells were cultured for three days, and the culture medium was replaced by the differentiation medium having the same composition, and the cells were further cultured for two days. The culturing was conducted at 37°C, 5% CO₂.

As a control in which differentiation is not induced, each of the KhES-1 cells seeded in the above two kinds of conditions was maintenance cultured in the same manner as described above, and after two days from the start of the maintenance culturing, the culture medium was replaced by a culture medium prepared by adding 4 ng/ml bFGF to MEF-CM (this point of time is day 0), then the cells were cultured for three days, and the culture medium was replaced by the culture medium having the same composition, and the cells were further cultured for two days. The culturing was conducted at 37°C, 2% CO₂.

The seeding condition and the composition of the culture medium used on day 0 or later are shown in Table 3.

**[Table 3]**

| | Seeding condition | Culture medium condition of day 0 or later | Note: |
|---|---|---|---|
| 3-1 | Cell mass | MEF-CM+4ng/ml bFGF | Control (Undifferentiated maintenance culturing) |
| 3-2 | Cell mass | MEF-CM + 10 ng/ml BMP4 + 1 µM A83-01 + 0.1 µM PD 173074 | Comparative Example |
| 3-3 | Cell mass | Basal differentiation medium B + 0.1 µM S26948 + 0.1 µM CGP 7930 + 10 nM SR 11237 + 1 nM retinoic acid | Production example |
| 3-4 | Single cell | MEF-CM+4ng/ml bFGF | Control (Undifferentiated maintenance culturing) |
| 3-5 | Single cell | MEF-CM + 10 ng/ml BMP4 + 1 µM A83-01 + 0.1 µM PD 173074 | Comparative Example |
| 3-6 | Single cell | Basal differentiation medium B + 0.1 µM S26948 + 0.1 µM CGP 7930 + 10 nM SR 11237 + 1 nM retinoic acid | Production example |

| | | | |
|---|---|---|---|
| Basal differentiation medium B: DMEM/F-12 medium supplemented with 10% FBS, 2 mM L-glutamine, 100 U/ml penicillin, 100 µg/ml streptomycin, 100 ng/ml BMP4, and 300 nM PD 173074 | | | |

On day 5 from day 0, total RNA was extracted from cells by using RNeasy Micro Kit (QIAGEN). The obtained total RNA was reverse transcribed by using SuperScript III (Invitrogen), and by real-time PCR using the obtained DNA as a template, TaqMan probe (Applied biosystems) and TaqMan Fast advanced master mix (Applied biosystems), the expression amount of Syncytin gene which is a marker gene of syncytiotrophoblast was examined. For the cells obtained in each culturing, the mRNA amount of Syncytin gene and the mRNA expression amount of GAPDH gene which is a house keeping gene were determined by real-time PCR. The value obtained by dividing the mRNA amount of Syncytin gene by the mRNA amount of GAPDH gene is defined as a correction value of mRNA amount of Syncytin gene, and taking the Syncytin gene mRNA amount correction value in cells that are seeded in the condition of 3-2 and cultured as 1, a Syncytin gene mRNA amount correction value in cells cultured in each of other conditions was shown as a relative expression amount. The operation from the cell culturing to measurement of mRNA amount was repeated three times. The result is shown in Fig. 3. In Fig. 3, a standard error of relative expression amount is indicated by an error bar. The relative expression amount having a statistically significant difference with respect to the relative expression amount of Comparative example 3-2 is marked with an asterisk (* means a P value of 0.01 or more and less than 0.05, and *** means a P value of less than 0.001). It was demonstrated that in the cells that were cultured in a differentiation medium containing S26948 which is a PPARγ agonist, CGP 7930 which is a GABABR positive allosteric modulator, SR 11237 which is an RXR agonist and retinoic acid which is an RAR agonist (3-3, 3-6), the relative expression amount of Syncytin gene increased in both the case where a cell mass was seeded and the case where singly dispersed cells were seeded, as compared with the Comparative examples (3-2, 3-5). It was demonstrated that the relative expression amount of Syncytin gene more increased in (3-6) in which singly dispersed cells were seeded than in (3-3) in which a cell mass was seeded.

### Example 5: Production of syncytiotrophoblasts differentiated from human ES cells

GCM1-GFP knock-in human ES cells prepared in Example 1 were maintenance cultured according to the method described in (1) of Example 2, and then subjected to a MEF removing operation.

### (1) Production example of the present invention

As Production example of the present invention, a mass of GCM1-GFP knock-in human ES cells having subjected to the MEF removing operation was dispersed into single cells by using TrypLE Express (Life Technologies), and the obtained cells were suspended in a culture medium of 150 µl so that 1.5 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 0.1 µM S26948 (PPAPγ agonist), 0.1 µM CGP 7930 (GABABR positive allosteric modulator) , 10 nM SR 11237 (RXR agonist), and 1 nM retinoic acid (RAR agonist) to Basal differentiation medium B (DMEM/F12 medium supplemented with 10% FBS, 2 mM L-glutamine, 100 U/ml penicillin, 100 µg/ml streptomycin, 100 ng/ml BMP4, and 300 nM PD 173074), and the cells were cultured at 37°C, 5% CO₂. After three days from the culture medium replacement, the culture medium was replaced by the differentiation medium having the same composition except that PD 173074 is not contained.

### (2) Comparative production example

As Comparative production example, a mass of knock-in human ES cells having subjected to the MEF removing operation was made into cell masses each consisting of several tens of cells by conducting pipetting 10 to 20 times with the use of a 1000 µl filter chip (NPPON Genetics), and the obtained cell mass was suspended in 150 µl of a culture medium so that about 1.5 × 10³ cells to about 4 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 10 ng/ml BMP4, 1 µM A83-01 and 0.1 µM PD 173074 to MEF-CM (described in Non-patent document 2), and cells were cultured at 37°C, 5% CO₂. After three days from the culture medium replacement, the culture medium was replaced by the differentiation medium having the same composition.

### (3) Cell observation

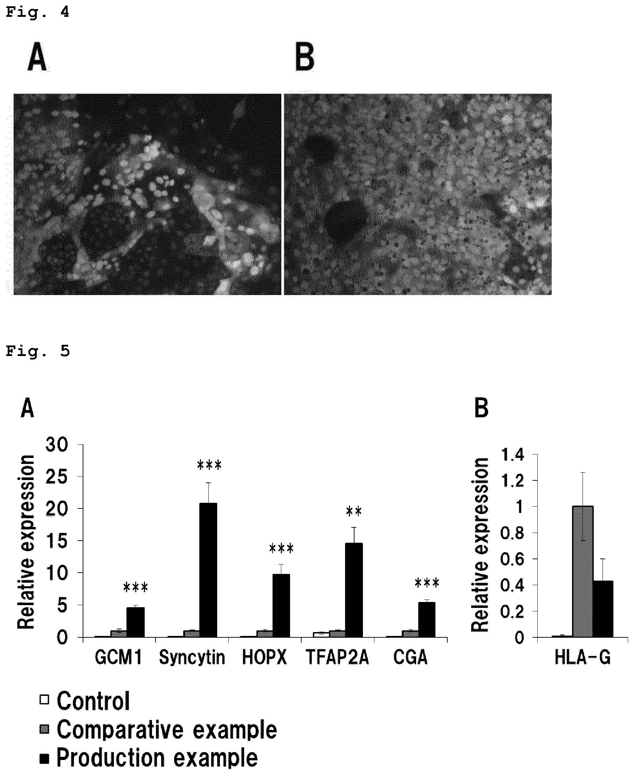

On day 5 of the culturing using the differentiation medium, cells were washed twice with PBS, and then fixed with 4% paraformaldehyde for 15 minutes. After subjecting the cells to a membrane permeabilizing treatment for 5 minutes by using PBS supplemented with 0.3% Triton X-100 (Wako), the cells were washed three times with PBS. Then after blocking the cells for 1 hour to 2 hours with 3% Bovine Serum Albumin, the cells were immunostained by using 5 µg/ml chicken anti-GFP antibody (Abcam), 2 µg/ml goat anti-chicken IgG antibody Alexa 488 (Life technologies) and 1µg/ml Hoechst 33342 (DOJINDO). The immunostained image of the obtained stained sample was observed under a fluorescence microscope. The result is shown in Fig. 4.

While the GFP positive cells indicating expression of GCM1 gene were observed at about 50% (Fig. 4A) in Comparative production example, the GFP positive cells occupied about 90% in the Production example of the present invention (Fig. 4B). Further, in Production example of the present invention, the fused cell-like morphology which is characteristic of syncytiotrophoblast was observed (Fig. 4B).

### Example 6: Production of syncytiotrophoblasts differentiated from human ES cells

According to the method described in (1) in Example 2, KhES-1 cells were maintenance cultured, and then subjected to the MEF removing operation.

### (1) Production example of the present invention

As Production example of the present invention, a mass of KhES-1 cells having been subjected to the MEF removing operation was dispersed into single cells by using TrypLE Express (Life Technologies), and the obtained cells were suspended in 150 µl of a culture medium so that 1.5 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 0.1 µM S26948 (PPAPγ agonist), 0.1 µM CGP 7930 (GABABR positive allosteric modulator) , 10 nM SR 11237 (RXR agonist), and 1 nM retinoic acid (RAR agonist) to Basal differentiation medium B (DMEM/F12 medium supplemented with 10% FBS, 2 mM L-glutamine, 100 U/ml penicillin, 100 µg/ml streptomycin, 100 ng/ml BMP4, and 300 nM PD 173074) (this point of time is day 0), and the cells were cultured at 37°C, 5% CO₂. After three days from the culture medium replacement, the culture medium was replaced by the differentiation medium having the same composition except that PD 173074 is not contained.

### (2) Comparative production example

As Comparative example, the mass of KhES cells having been subjected to the MEF removing operation was made into cell masses each consisting of several tens of cells by conducting pipetting 10 to 20 times with the use of a 1000 µl filter chip (NPPON Genetics), and the obtained cell mass was suspended in 150 µl of a culture medium so that about 1.5 × 10³ cells to about 4 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 10 ng/ml BMP4, 1 µM A83-01 and 0.1 µM PD 173074 to MEF-CM (this point of time is day 0) (described in Non-patent document 2), and cells were cultured at 37°C, 5% CO₂. After three days from the culture medium replacement, the culture medium was replaced by the differentiation medium having the same composition.

### (3) Control (Undifferentiated maintenance culturing)

As a control in which differentiation is not induced, the mass of KhES cells having been subjected to the MEF removing operation was made into cell masses each consisting of several tens of cells by conducting pipetting 10 to 20 times with the use of a 1000 µl filter chip (NPPON Genetics), and the obtained cell mass was suspended in 150 µl of a culture medium so that about 1.5 × 10³ cells to about 4 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a culture medium prepared by adding 4ng/ml bFGF to MEF-CM (this point of time is day 0), then the cells were cultured for three days, and the culture medium was replaced by the culture medium having the same composition, and the cells were further cultured for two days. The culturing was conducted at 37°C, 2% CO₂.

### (4) Gene expression analysis

On day 5 from day 0, total RNA was extracted from cells by using RNeasy Micro Kit (QIAGEN) . The obtained total RNA was reverse transcribed by using SuperScript III (Invitrogen), and by real-time PCR using the obtained DNA as a template, TaqMan probe (Applied biosystems) and TaqMan Fast advanced master mix (Applied biosystems), the expression amounts of GCM1 gene, Syncytin gene, HOPX gene, TFAP2A gene and CGA gene that are known as marker genes of syncytiotrophoblast, and HLA-G gene that is known as a marker gene of extravillous trophoblast which is a placenta cell of a different cell line from syncytiotrophoblast were examined. For the cells obtained in each culturing, the mRNA expression amount of each syncytiotrophoblast marker gene, the mRNA expression amount of HLA-G gene, and the mRNA expression amount of GAPDH gene which is a house keeping gene were determined by real-time PCR. The value obtained by dividing the mRNA amount of each syncytiotrophoblast marker gene by the mRNA amount of GAPDH gene is defined as a correction value of mRNA amount of the syncytiotrophoblast marker gene, and taking the syncytiotrophoblast marker gene mRNA amount correction value in cells obtained in Comparative production example (described in (2)) as 1, a syncytiotrophoblast marker gene mRNA amount correction value in cells obtained in Production example of the present invention (described in (1)) or in Control (described in (3)) was shown as a relative expression amount. Also, the mRNA amount of HLA-G gene was subjected to the same data processing, and shown as a relative expression amount. The operation from the cell culturing to measurement of mRNA amount was repeated three times . The result is shown in Fig. 5. In Fig. 5, the relative expression amount in cells of Control (undifferentiated maintenance culturing) is indicated by a white bar graph, the relative expression amount in cells of Comparative example is indicated by a gray bar graph, and the relative expression amount in cells of Production example of the present invention is indicated by a black bar graph. A standard error of relative expression amount is indicated by an error bar. The relative expression amount having a statistically significant difference with respect to the relative expression amount of Comparative production example is marked with asterisks (** means a P value of 0.001 or more and less than 0.01, and *** means a P value of less than 0.001). In the cells obtained in Production example of the present invention, the relative expression amount of each syncytiotrophoblast marker gene significantly increased in the range of 5 times to 20 times (Fig. 5A), and the relative expression amount of a extravillous trophoblast marker gene decreased to about 40% (Fig. 5B) compared with the cells obtained in Comparative production example.

This result indicates that Production method of the present invention enables production of syncytiotrophoblasts from human ES cells with high efficiency.

### Example 7: Production of syncytiotrophoblasts differentiated from human ES cells

According to the method described in (1) in Example 2, KhES-1 cells were maintenance cultured, and then subjected to the MEF removing operation. The mass of KhES-1 cells having been subjected to the MEF removing operation was dispersed into single cells by using TrypLE Express (Life Technologies), and the obtained cells were suspended in 150 µl of a culture medium so that 1.5 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. As a culture medium at this time, a culture medium in which bFGF and Y-27632 are added at 10 g/ml and 20 µM, respectively to MEF-CM was used.

### (1) Control

After two days from the start of the maintenance culturing, the culture medium was replaced by Basal differentiation medium B (DMEM/F12 medium supplemented with 10% FBS, 2 mM L-glutamine, 100 U/ml penicillin, 100 µg/ml streptomycin, 100 ng/ml BMP4, and 300 nM PD 173074) (this point of time is day 0 of the differentiation culturing), and the cells were cultured for five days at 37°C, 5% CO₂ (Control) . After three days from the culture medium replacement, the culture medium was replaced by the differentiation medium having the same composition except that PD 173074 is not contained.

### (2) Production example of the present invention

After two days from the start of the maintenance culturing, the culture medium was replaced by Basal differentiation medium B or a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal differentiation medium B (hereinafter, sometimes referred to as Differentiation medium B) (this point of time is day 0 of the differentiation culturing), and the cells were cultured for five days at 37°C, 5% CO₂.

For part of wells for which medium replacement by Basal differentiation medium B was conducted after two days from the start of the maintenance culturing, replacement by Differentiation medium B was conducted on day 1, 2, 3 or 4 from the start of the culturing using Basal differentiation medium B, and the culturing was continued until day 5.

For part of wells for which medium replacement by Differentiation medium B was conducted after two days from the start of the maintenance culturing, replacement by Basal differentiation medium B was conducted on day 1, 2, 3 or 4 from the start of the culturing using Differentiation medium B, and the culturing was continued until day 5.

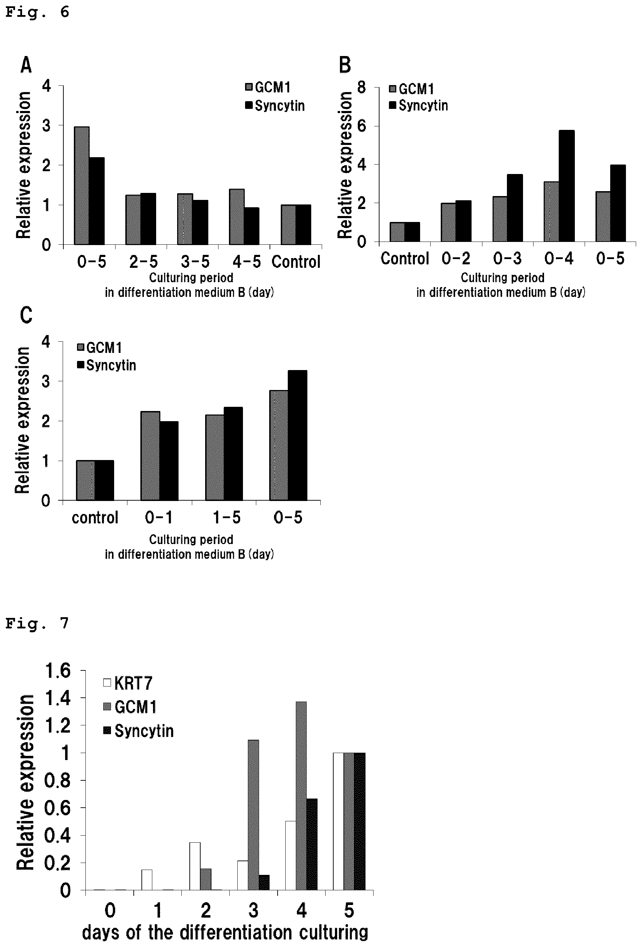

That is, in the period from day 0 to day 5 of the differentiation culturing, from day 2 to day 5 of the differentiation culturing, from day 3 to day 5 of the differentiation culturing, or from day 4 to day 5 of the differentiation culturing (Fig. 6A), the period from day 0 to day 2 of the differentiation culturing, from day 0 to day 3 of the differentiation culturing, from day 0 to day 4 of the differentiation culturing, or from day 0 to day 5 of the differentiation culturing (Fig. 6B), or the period from day 0 to day 1 of the differentiation culturing, from day 1 to day 5 of the differentiation culturing, or from day 0 to day 5 of the differentiation culturing (Fig. 6C), the culturing was conducted using Differentiation medium B, and in other periods, the culturing was conducted using Basal differentiation medium B.

In any case, from day 3 of the differentiation culturing, the culture medium having the same composition as described above except that PD 173074 is not contained was used.

### (3) Gene expression analysis

On day 5 of the differentiation culturing, total RNA was extracted from cells using RNeasy Micro Kit(QIAGEN). The obtained total RNA was reverse transcribed by using SuperScript III (Invitrogen), and by real-time PCR using the obtained DNA as a template, TaqMan probe (Applied biosystems) and TaqMan Fast advanced master mix (Applied biosystems), the expression amounts of GCM1 gene and Syncytin gene which are marker genes of syncytiotrophoblast were examined. The mRNA amounts of GCM1 gene, Syncytin gene and GAPDH gene which is a house keeping gene were determined by real-time PCR. The value obtained by dividing the mRNA amount of Syncytin gene by the mRNA amount of GAPDH gene is defined as a correction value of mRNA amount of Syncytin gene, and taking the Syncytin gene mRNA amount correction value in cells that are obtained in Control (described in (1)) as 1, a Syncytin gene mRNA amount correction value in cells obtained in Production example of the present invention (described in (2)) was shown as a relative expression amount. Also, the mRNA amount of GCM1 gene was subjected to the same data processing, and shown as a relative expression amount. The result is shown in Fig. 6. In Fig. 6, Production example of the present invention is shown by specifying with a culturing period (day) in Differentiation medium B, and the relative expression amount of GCM1 gene is indicated by a gray bar graph, and the relative expression amount of Syncytin gene is indicated by a black bar graph.

When the culturing with Differentiation medium B was started on day 2 or later from the start of the differentiation culturing, significant increase was not observed in the expression amounts of GCM1 gene and Syncytin gene which are syncytiotrophoblast marker genes (Fig. 6A: 2-5, 3-5 and 4-5) in comparison with the case where the culturing was conducted for 5 days only with Basal differentiation medium B (Control) . When the culturing with Differentiation medium B was started before day 2 of the differentiation culturing, the expression amounts of GCM1 gene and Syncytin gene increased twice or more compared with Control, and the expression amounts of GCM1 gene and Syncytin gene were higher than that in Control even when the replacement by Basal differentiation medium B was conducted before day 5 of the differentiation culturing (Figs. 6B and C). When the cells were cultured in a differentiation medium in which S26948 which is a PPAPγ agonist, CGP 7930 which is a GABABR positive allosteric modulator, SR 11237 which is an RXR agonist, and retinoic acid which is an RAR agonist are contained in Basal differentiation medium B (Differentiation medium B) in the entire period of the differentiation culturing of five days, the expression amounts of GCM1 gene and Syncytin gene increased about twice to four times as compared with Control (Figs. 6A, B and C) .

### Example 8: Production of trophoblasts and syncytiotrophoblasts differentiated from human ES cells

According to the method described in (1) in Example 2, KhES-1 cells were maintenance cultured, and then subjected to the MEF removing operation. The mass of KhES-1 cells having been subjected to the MEF removing operation was dispersed into single cells by using TrypLE Express (Life Technologies), and the obtained cells were suspended in 150 µl of a culture medium so that 1.5 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. As a culture medium at this time, a culture medium in which 10 ng/ml bFGF and 20 µM Y-27632 are added to MEF-CM was used. After two days from the start of the maintenance culturing, the culture medium was replaced by a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal differentiation medium B (DMEM/F12 medium supplemented with 10% FBS, 2 mM L-glutamine, 100 U/ml penicillin, 100 µg/ml streptomycin, 100 ng/ml BMP4, 300 nM PD 173074) (this point of time is day 0 of the differentiation culturing), and the cells were cultured for five days at 37°C, 5% CO₂. On day 3 of the differentiation culturing, the culture medium was replaced by the differentiation medium having the same composition except that PD 173074 is not contained.

On days 0, 1, 2, 3, 4 and 5 of the differentiation culturing, total RNA was extracted from cells using RNeasy Micro Kit (QIAGEN). The obtained total RNA was reverse transcribed by using SuperScript III (Invitrogen), and by real-time PCR using the obtained DNA as a template, TaqMan probe (Applied biosystems) and TaqMan Fast advanced master mix (Applied biosystems), the expression amounts of trophoblast marker KRT7 gene, and GCM1 gene and Syncytin gene which are marker genes of syncytiotrophoblast were examined. The mRNA amounts of KRT7 gene, GCM1 gene, Syncytin gene and GAPDH gene which is a house keeping gene were determined by real-time PCR. The value obtained by dividing the mRNA amount of Syncytin gene by the mRNA amount of GAPDH gene is defined as a correction value of mRNA amount of Syncytin gene, and taking the Syncytin gene mRNA amount correction value in cells that are obtained on day 5 of the differentiation culturing as 1, a Syncytin gene mRNA amount correction value in cells obtained on day 0, 1, 2, 3 or 4 of the differentiation culturing was shown as a relative expression amount. Also, the mRNA amounts of KRT7 gene and GCM1 gene were subjected to the same data processing, and shown as relative expression amounts. The result is shown in Fig. 7. In Fig. 7, the relative expression amount of KRT7 gene is indicated by a white bar graph, the relative expression amount of GCM1 gene is indicated by a gray bar graph, and the relative expression amount of Syncytin gene is indicated by a black bar graph.

On day 0 of the differentiation culturing, expression of none of KRT7 gene, GCM1 gene and Syncytin gene were observed. The expression amount of KRT7 gene was detected from day 1 of the differentiation culturing, and peaked on day 5 of the differentiation culturing. The expression amount of GCM1 gene started increasing on day 2 of the differentiation culturing, and peaked on day 4 of the differentiation culturing. The expression amount of Syncytin gene started increasing on day 3 of the differentiation culturing, and peaked on day 5 of the differentiation culturing.

In Example 7, it was suggested that the time "before day 2 of the differentiation culturing" "when the culturing with Differentiation medium B is started before day 2 of the differentiation culturing" in which the expression amounts of GCM1 gene and Syncytin gene increased compared with Control corresponds to the time "before appearance of cells expressing GCM1 mRNA in the culture".

### Example 9: Production of syncytiotrophoblasts differentiated from human ES cells

GCM1-GFP knock-in human ES cells prepared in Example 1 were maintenance cultured according to the method described in (1) of Example 2, and then subjected to a MEF removing operation.

The mass of GCM1-GFP knock-in human ES cells having been subjected to the MEF removing operation was dispersed into single cells by using TrypLE Express (Life Technologies), and the obtained cells were suspended in a culture medium of 150 µl so that 1.5 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. As a culture medium at this time, a culture medium prepared by adding 10 ng/ml bFGF and 20 µM Y-27632 to MEF-CM was used.

After two days from the start of the maintenance culturing, the culture medium was replaced by a differentiation medium prepared by adding retinoic acid (RAR agonist) in 1 nM, 10 nM, 100 nM, or 1000 nM to Basal differentiation medium B (DMEM/F12 medium supplemented with 10% FBS, 2 mM L-glutamine, 100 U/ml penicillin, 100 µg/ml streptomycin, 100 ng/ml BMP4, and 300 nM PD 173074), or by a differentiation medium prepared by adding DMSO in a concentration of 0.01% to Basal differentiation medium B, and cells were cultured at 37°C, 5% CO₂. After three days from the culture medium replacement, the culture medium was replaced by the culture medium having the same composition except that PD 173074 is not contained.

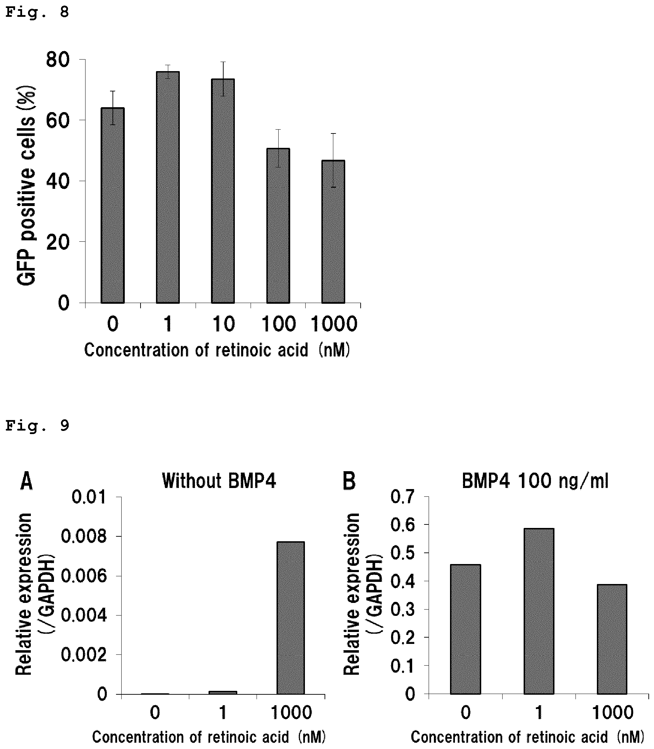

On day 5 of the culture using the differentiation medium, cells were washed twice with PBS (Invitrogen), and fixed with 4% paraformaldehyde (Wako) for 15 minutes. After subjecting the cells to a membrane permeabilizing treatment for 5 minutes by using PBS supplemented with 0.3% Triton X-100 (Wako), the cells were washed three times with PBS. Then after blocking the cells for 1 hour to 2 hours with 3% Bovine Serum Albumin, the cells were immunostained by using 5 µg/ml chicken anti-GFP antibody (Abcam), 2 µg/ml goat anti-chicken IgG antibody Alexa 488 (Life technologies) and 1 µg/ml Hoechst 33342 (DOJINDO). For the obtained stained sample, the percentage of the GFP positive cells was quantified by using a fully automatic image analyzer Arrayscan (Thermo Scientific). The operation from the cell culturing to measurement of GFP positive cells was repeated three times. The result is shown in Fig. 8. A standard error of the percentage of GFP positive cells is indicated by an error bar.

When a differentiation medium supplemented with 1 nM or 10nM retinoic acid was used, the percentage of GFP positive cells increased about 10% compared with the case where a differentiation medium not supplemented with retinoic acid was used (Control). On the other hand, when a differentiation medium supplemented with 100 nM or more retinoic acid was used, the percentage of GFP positive cells decreased about 10% or more compared with Control.

### Example 10: Production of syncytiotrophoblasts differentiated from human ES cells

According to the method described in (1) in Example 2, KhES-1 cells were maintenance cultured, and then subjected to the MEF removing operation.

The mass of KhES-1 cells having been subjected to the MEF removing operation was dispersed into single cells by using TrypLE Express (Life Technologies), and the obtained cells were suspended in 150 µl of a culture medium so that 1.5 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. As a culture medium at this time, a culture medium prepared by adding 10 ng/ml bFGF and 20 µM Y-27632 to MEF-CM was used.

After two days from the start of the maintenance culturing, the culture medium was replaced by a DMEM/F12 medium supplemented with 10% FBS, 2 mM L-glutamine, 100 U/ml penicillin and 100 µg/ml streptomycin (hereinafter, referred to as Basal medium C); a culture medium prepared by adding 1 nM or 1000 nM retinoic acid to Basal medium C; a differentiation medium prepared by adding 100ng/ml BMP4 to Basal medium C; a differentiation medium prepared by adding 100 ng/ml BMP4 and 1 nM retinoic acid to Basal medium C; or a differentiation medium prepared by adding 100 ng/ml BMP4 and 1000 nM retinoic acid to Basal medium C (this point of time is day 0), and cells were cultured at 37°C, 5% CO₂. After three days from the culture medium replacement, the culture medium was replaced by the culture medium having the same composition, and cells were cultured for another two days.

On day 5 from day 0, total RNA was extracted from cells by using RNeasy Micro Kit (QIAGEN) . The obtained total RNA was reverse transcribed by using SuperScript III (Invitrogen), and by real-time PCR using the obtained DNA as a template, TaqMan probe (Applied biosystems) and TaqMan Fast advanced master mix (Applied biosystems), the expression amount of Syncytin gene which is a marker gene of syncytiotrophoblast was examined. For the cells obtained in each culturing, the mRNA expression amounts of Syncytin gene and GAPDH gene which is a house keeping gene were determined by real-time PCR. The value obtained by dividing the mRNA amount of Syncytin gene by the mRNA amount of GAPDH gene was shown as a relative expression amount of Syncytin gene. The result is shown in Fig. 9. Fig. 9A shows the result when a culture medium not supplemented with BMP4 is was used, and Fig. 9B shows the result when a differentiation medium supplemented with 100 ng/ml BMP4 was used.

In the absence of BMP4, the relative expression amount of Syncytin gene slightly increased when the culture medium supplemented with 1000 nM retinoic acid was used, compared with the case where the culture medium not supplemented with retinoic acid was used (Fig. 9A). On the other hand, in the presence of 100 ng/ml BMP4, the relative expression amount of Syncytin gene increased when a differentiation medium supplemented with 1 nM retinoic acid was used, and the relative expression amount of Syncytin gene decreased when a differentiation medium supplemented with 1000 nM retinoic acid was used, in comparison with the case where a culture medium not supplemented with retinoic acid was used(Fig. 9B).

### Example 11: Production of trophoblasts and syncytiotrophoblasts differentiated from human ES cells, and cell layer permeability test using these

According to the method described in (1) in Example 2, human ES cells are maintenance cultured, and then subjected to the MEF removing operation.

The mass of human ES cells having been subjected to the MEF removing operation is dispersed into single cells by using TrypLE Express (Life Technologies), and the obtained cells are suspended in 150 µl of a culture medium so that 1.5 × 10³ cells are contained per one well, and seeded on a 96-well culture plate coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM is used.

After two days from the start of the maintenance culturing, the culture medium is replaced by a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal differentiation medium B (DMEM/F12 medium supplemented with 10% FBS, 2 mM L-glutamine, 100 U/ml penicillin, 100 µg/ml streptomycin, 100 ng/ml BMP4, and 300 nM PD 173074) (this point of time is day 0), and the cells are cultured for one day to two days at 37°C, 5% CO₂ to obtain a culture containing trophoblasts differentiated from the human ES cells. For the obtained culture, existence of a cell in which mRNA encoding Cytokeratin 7 is expressed, but mRNA encoding Syncytin is not expressed is examined as is necessary.

The cells obtained in the above culturing are cultured on a cell culture insert (Corning, Cat.3470, membrane pore size 0.4 µm) for one day to four days at 37°C, 5% CO₂. As a culture medium, a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal differentiation medium B (DMEM/F12 medium supplemented with 10% FBS, 2 mM L-glutamine, 100 U/ml penicillin, 100 µg/ml streptomycin, 100 ng/ml BMP4, and 300 nM PD 173074) is used. On day 3 or later from day 0, the differentiation medium having the same composition as described above except that PD 173074 is not contained is used. Appearance of syncytiotrophoblasts differentiated from the human ES cells is examined as necessary.

A permeability test of a test substance is conducted by referring to a placenta permeability test method as described, for example, in "Poulsen, M. et al. Toxicology in Vitro 2009, 23, 1380-1386".

The electric resistance (Ω) of the cell layer formed on the cell culture insert is measured by using a Millicell ERS-2 (Millipore), and from the obtained measurement value, the electric resistance of the blank in which cells are not seeded is subtracted, and the resultant value is multiplied with the area of the membrane filter (cm²) to determine a transepithelial electric resistance (TEER, Ω•cm²). A cell layer that forms a tight junction showing a TEER value of 35 or more is used for the permeability test.

The culture medium in the insert and in the multiple well plate is replaced by Hanks' Balanced Salt Solution (HBSS) (no phenol red, Life Technologies), and incubated for 30 minutes to 45 minutes at 37°C 5% CO₂. The culture medium in the insert (apical side) is replaced by HBSS containing a test substance, and cultured at 37°C, 0% to 5% CO₂ under stirring at 70 rpm. After 30 minutes, 60 minutes, 90 minutes and 120 minutes, 100µl of the culture medium is collected from inside the multiple well plate, and the same amount of HBSS is added to the culture medium. By quantifying the test substance in the culture medium in the multiple well plate by using, for example, a fluorescence plate reader (PerkinElmer) or LC-MS, the permeability of the test substance in the cell layer is evaluated.

### Example 12: Production of trophoblasts and syncytiotrophoblasts differentiated from human iPS cells

According to the method described in (1) in Example 2, human iPS cell line 201B7 (available from Center for iPS Cell Research and Application, Kyoto University) is maintenance cultured, and then subjected to the MEF removing operation.

The mass of KhES-1 cells having been subjected to the MEF removing operation is dispersed into single cells by using TrypLE Express (Life Technologies), and the obtained cells are suspended in 150 µl of a culture medium so that 1.5 × 10³ cells are contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. As a culture medium at this time, a culture medium prepared by adding 10 ng/ml bFGF and 20 µM Y-27632 to MEF-CM is used.

After two days from the start of the maintenance culturing, the culture medium is replaced by a differentiation medium prepared by adding either one of 0.1 µM S26948 (PPAPγ agonist), 0.1 µM CGP 7930 (GABABR positive allosteric modulator), 10 nM SR 11237 (RXR agonist), or 1 nM retinoic acid (RAR agonist) to Basal medium B (DMEM/F12 medium supplemented with 10% FBS, 2 mM L-glutamine, 100 U/ml penicillin, 100 µg/ml streptomycin, 100 ng/ml BMP4, 300 nM PD 173074), or a differentiation medium supplemented with two or more selected from the group consisting of 0.1 µM S26948, 0.1 µM CGP 7930, 10 nM SR 11237 and 1 nM retinoic acid (RAR agonist) (this point of time is day 0), and the cells are cultured for one day to two days at 37°C, 5% CO₂ to obtain a culture containing trophoblasts differentiated from the human iPS cells. For the obtained culture, existence of a cell in which mRNA encoding Cytokeratin 7 is expressed, but mRNA encoding Syncytin is not expressed is examined as is necessary.

The culturing from day 0 using the differentiation medium is continued, and a culture containing syncytiotrophoblasts differentiated from the human iPS cells is obtained. On day 3 or later from day 0, the differentiation medium having the same composition as described above except that PD 173074 is not contained is used.

### Example 13: Production of trophoblasts and syncytiotrophoblasts differentiated from human ES cells, and cell layer permeability test using these

According to the method described in (1) in Example 2, KhES-1 cells were maintenance cultured, and then subjected to the MEF removing operation.

The mass of KhES-1 cells having been subjected to the MEF removing operation was dispersed into single cells by using TrypLE Express (Life Technologies), and the obtained cells were suspended in 10 ml of a culture medium so that 1.5 × 10⁵ cells were contained per one well, and seeded on a 100 mm cell culture dish (BD Falcon) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal differentiation medium B (DMEM/F12 medium supplemented with 10% FBS, 2 mM L-glutamine, 100 U/ml penicillin, 100 µg/ml streptomycin, 100 ng/ml BMP4, and 300 nMPD 173074) supplemented with 20µM Y-27632 (this point of time is day 0), and the cells were cultured for two days at 37°C, 5% CO₂ to obtain a culture containing trophoblasts differentiated from the KhES-1 cells.

The cells obtained in the culturing were washed twice with PBS while they were adhered to the cell culture dish, and then PBS supplemented with 2mg/ml collagenaseIV(Invitrogen) and 20µg/ml DNaseI(Roche) was added to the dish, and the dish was incubated for 5 minutes at 37°C, 5% CO₂. TrypLE Express (Life Technologies) was further added to the dish, and the dish was incubated for 5 minutes at 37°C, 5% CO₂. After adding to the dish a DMEM/F-12 medium supplemented with 10% FBS (Corning), 2mM L-glutamine, and 100 U/ml penicillin, 100 µg/ml streptomycin (Penicillin-Streptomycin Mixed Solution, Nacalai Tesque), the cells were peeled off by pipetting, and the culture medium containing cells were collected, and centrifuged (1000 rpm, 5 minutes). After removing the supernatant from the centrifuged culture, a differentiation medium prepared by adding 0.1 µM S26948 (PPAPγ agonist), 0.1 µM CGP 7930 (GABABR positive allosteric modulator), 10 nM SR 11237 (RXR agonist), 1 nM retinoic acid (RAR agonist), 2 µM Caspase Inhibitor Z-VAD-FMK (Promega), 10 ng/ml HGF (R&D) and 10 µM SB203580 (p38 MAPK inhibitor, Abcam) to Basal differentiation medium B (DMEM/F12 medium supplemented with 10% FBS, 2 mM L-glutamine, 100 U/ml penicillin, 100 µg/ml streptomycin, 100ng/ml BMP4 , and 300nM PD 173074) (hereinafter, referred to as Basal differentiation medium C) was added to the deposit to suspend the cells, and then the obtained cell suspension was seeded on a cell culture insert (Corning, Cat.3470, membrane pore size 0.4 µm) coated with 0.1% gelatin (Sigma-Aldrich), and cultured at 37°C, 5% CO₂. As a culture medium, Basal differentiation medium C was used. After one day from the seeding to the cell culture insert, the culture medium was replaced by the differentiation medium having the same composition except that SB203580 is not contained, and the cells were cultured at 37°C, 5% CO₂.

After two days from the seeding to the cell culture insert, the electric resistance (Ω) of the cell layer formed on the cell culture insert was measured by using a Millicell ERS-2 (Millipore), and from the obtained measurement value, the electric resistance of the blank in which cells are not seeded was subtracted, and the resultant value was multiplied with the area of the membrane filter (cm²) to determine a transepithelial electric resistance (TEER, Ω•cm²). A cell layer that forms a tight junction showing a TEER value of 250 or more was used for the permeability test.

The culture medium in the insert and in the multiple well plate was replaced by Hanks' Balanced Salt Solution (HBSS) (no phenol red, Life Technologies), and incubated for 30 minutes to 45 minutes at 37°C 5% CO₂. The culture medium in the insert (apical side) is replaced by HBSS containing a test substance, and cultured at 37°C under stirring at 300 rpm.

After 120 minutes from addition of the test substance, 100 µl of HBSS containing the permeated test substance was collected from inside the multiple well plate, and the same amount of HBSS was added to the collected HBSS. By quantifying the test substance in the culture medium in the multiple well plate by LC-MS, the permeability of the test substance in the cell layer was evaluated. A value obtained by dividing the permeation amount of each test substance by the permeation amount of antipyrine is shown as an in vitro relative permeation amount in Table 4. A relative permeation amount of each test substance obtained from the permeability test by the placenta perfusion method using term placenta was excerpted from "Li, H. et al. Arch. Toxicol. 2013, 87, 1661-1669" and shown as an ex vivo relative permeation amount (literature value) in Table 4.

**[Table 4]**

| Name of test substance | In vitro relative permeation amount | Ex vivo relative permeation amount (literature value) |
|---|---|---|
| Antipyrine | 1 | 1 |
| Caffeine | 1.4 | 1.5 |
| Lidocaine | 0.96 | 0.83 |
| Indomethacin | 0.72 | 0.66 |
| Levofloxacin | 0.18 | 0.33 |
| Digoxin | 0.24 | 0.13 |

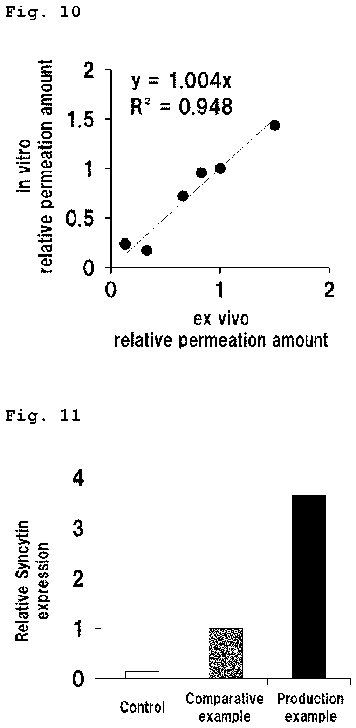

Correlation between the in vitro relative permeation amount and ex vivo relative permeation amount of each test substance was examined. The result is shown in Fig. 10. High correlation was shown as seen from the square of the correlation coefficient between the in vitro relative permeation amount and ex vivo relative permeation amount of each test substance of 0.948 (Fig. 10).

This result revealed that the cell layer permeability of a test substance in placenta cells can be assayed by using the cells produced by Production method of the present invention.

### Example 14: Production of syncytiotrophoblasts differentiated from human iPS cells

According to the method described in (1) in Example 2, human iPS cell line 201B7 (available from Center for iPS Cell Research and Application, Kyoto University) was maintenance cultured, and then subjected to the MEF removing operation.

### (1) Production example of the present invention

As Production example of the present invention, a mass of human iPS cells having been subjected to the MEF removing operation was dispersed into single cells by using TrypLE Express (Life Technologies), and the obtained cells were suspended in 150 µl of a culture medium so that 1.5 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal medium B (DMEM/F12 medium supplemented with 10% FBS, 2 mM L-glutamine, 100 U/ml penicillin, 100 µg/ml streptomycin, 100 ng/ml BMP4, 300 nM PD 173074) supplemented with 20 µM Y-27632 (this point of time is day 0), and the cells were cultured at 37°C, 5% CO₂. After three days from the culture medium replacement, the culture medium was replaced by the differentiation medium having the same composition except that PD 173074 is not contained.

### (2) Comparative production example

As Comparative example, a mass of 201B7 cells having been subjected to the MEF removing operation was made into cell masses each consisting of several tens of cells by conducting pipetting 10 to 20 times with the use of a 1000 µl filter chip (NPPON Genetics), and the obtained cell mass was suspended in 150 µl of a culture medium so that about 1.5 × 10³ cells to about 4 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 10 ng/ml BMP4, 1 µM A83-01 and 0.1 µM PD 173074 to MEF-CM (this point of time is day 0) (described in Non-patent document 2), and cells were cultured at 37°C, 5% CO₂. After three days from the culture medium replacement, the culture medium was replaced by the differentiation medium having the same composition.

### (3) Control (Undifferentiated maintenance culturing)

As a control in which differentiation is not induced, the mass of 201B7 cells having been subjected to the MEF removing operation was made into cell masses each consisting of several tens of cells by conducting pipetting 10 to 20 times with the use of a 1000 µl filter chip (NPPON Genetics), and the obtained cell mass was suspended in 150 µl of a culture medium so that about 1.5 × 10³ cells to about 4 × 10³ cells were contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a culture medium prepared by adding 4ng/ml bFGF to MEF-CM (this point of time is day 0), then the cells were cultured for three days, and the culture medium was replaced by the culture medium having the same composition, and the cells were further cultured for two days. The culturing was conducted at 37°C, 2% CO₂.

### (4) Gene expression analysis

On day 6 (day 6) from day 0, total RNA was extracted from cells by using RNeasy Micro Kit (QIAGEN). The obtained total RNA was reverse transcribed by using SuperScript III (Invitrogen), and by real-time PCR using the obtained DNA as a template, TaqMan probe (Applied biosystems) and TaqMan Fast advanced master mix (Applied biosystems), the expression amount of Syncytin gene which is known as a marker gene of syncytiotrophoblast was examined. For the cells obtained in each culturing, the mRNA expression amount of Syncytin gene and the mRNA expression amount of GAPDH gene which is a house keeping gene were determined by real-time PCR. The value obtained by dividing the mRNA amount of Syncytin gene by the mRNA amount of GAPDH gene is defined as a correction value of mRNA amount of Syncytin gene, and taking the Syncytin gene mRNA amount correction value in cells that are obtained in Comparative production example (described in (2)) as 1, a Syncytin gene mRNA amount correction value in cells obtained in Production example of the present invention (described in (1)) or Control (described in (3)) was shown as a relative expression amount. The result is shown in Fig. 11. In Fig. 11, the relative expression amount in cells of Control (undifferentiated maintenance culturing) is indicated by a white bar graph, the relative expression amount in cells of Comparative example is indicated by a gray bar graph, and the relative expression amount in cells of Production example of the present invention is indicated by a black bar graph. In the cells obtained in Production example of the present invention, the relative expression amount of Syncytin gene increased about 3.7 times compared with the cells obtained in Comparative production example (Fig. 11).

This result indicates that Production method of the present invention enables production of syncytiotrophoblasts from human iPS cells with high efficiency.

### Example 15: Production of trophoblasts and syncytiotrophoblasts differentiated from human iPS cells, and cell layer permeability test using these

According to the method described in (1) in Example 2, human iPS cell line 201B7 was maintenance cultured, and then subjected to the MEF removing operation.

The mass of 201B7 cells having been subjected to the MEF removing operation was dispersed into single cells by using TrypLE Express (Life Technologies), and the obtained cells were suspended in 10 ml of a culture medium so that 1.5 × 10⁵ cells were contained per one well, and seeded on a 100 mm cell culture dish (BD Falcon) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal differentiation medium B (DMEM/F12 medium supplemented with 10% FBS, 2 mM L-glutamine, 100 U/ml penicillin, 100 µg/ml streptomycin, 100 ng/ml BMP4, and 300 nM PD 173074) supplemented with 20µM Y-27632 (this point of time is day 0), and the cells were cultured for two days at 37°C, 5% CO₂ to obtain a culture containing trophoblasts differentiated from the 201B7 cells.

The cells obtained in the culturing were washed twice with PBS while they were adhered to the cell culture dish, and then PBS supplemented with 2mg/ml collagenaseIV(Invitrogen) and 20µg/ml DNaseI(Roche) was added to the dish, and the dish was incubated for 5 minutes at 37°C, 5% CO₂. TrypLE Express (Life Technologies) was further added to the dish, and the dish was incubated for 5 minutes at 37°C, 5% CO₂. After adding to the dish a DMEM/F-12 medium supplemented with 10% FBS (Corning), 2 mM L-glutamine, and 100 U/ml penicillin, 100 µg/ml streptomycin (Penicillin-Streptomycin Mixed Solution, Nacalai Tesque), the cells were peeled off by pipetting, and the culture medium containing cells were collected, and centrifuged (1000 rpm, 5 minutes). After removing the supernatant from the centrifuged culture, a differentiation medium prepared by adding 0.1 µM S26948 (PPAPγ agonist), 0.1 µM CGP 7930 (GABABR positive allosteric modulator), 10 nM SR 11237 (RXR agonist), 1 nM retinoic acid (RAR agonist), 2 µM Caspase Inhibitor Z-VAD-FMK (Promega), 10 ng/ml HGF (R&D) and 10 µM SB203580 (p38 MAPK inhibitor, Abcam) to Basal differentiation medium B (DMEM/F12 medium supplemented with 10% FBS, 2 mM L-glutamine, 100 U/ml penicillin, 100 µg/ml streptomycin, 100ng/ml BMP4, and 300nM PD 173074) (hereinafter, referred to as Basal differentiation medium C) was added to the deposit to suspend the cells, and then the obtained cell suspension was seeded on a cell culture insert (Corning, Cat.3470, membrane pore size 0.4 µm) coated with 0.1% gelatin (Sigma-Aldrich), and cultured at 37°C, 5% CO₂. As a culture medium, Basal differentiation medium C was used. After one day from the seeding to the cell culture insert, the culture medium was replaced by the differentiation medium having the same composition except that SB203580 is not contained, and the cells were cultured at 37°C, 5% CO₂.

After four days from the seeding to the cell culture insert, the electric resistance (Ω) of the cell layer formed on the cell culture insert was measured by using a Millicell ERS-2 (Millipore), and from the obtained measurement value, the electric resistance of the blank in which cells are not seeded was subtracted, and the resultant value was multiplied with the area of the membrane filter (cm²) to determine a transepithelial electric resistance (TEER, Ω•cm²). A cell layer that forms a tight junction showing a TEER value of 250 or more was used for the permeability test.

The culture medium in the insert and in the multiple well plate was replaced by Hanks' Balanced Salt Solution (HBSS) (no phenol red, Life Technologies), and incubated for 30 minutes to 45 minutes at 37°C 5% CO₂. The culture medium in the insert (apical side) is replaced by HBSS containing a test substance, and cultured at 37°C under stirring at 300 rpm.

After 120 minutes from addition of the test substance, 100 µl of HBSS containing the test substance was collected from inside the multiple well plate, and the same amount of HBSS was added to the collected HBSS. By quantifying the test substance in the culture medium in the multiple well plate by LC-MS, the permeability of the test substance in the cell layer was evaluated. A value obtained by dividing the permeation amount of each test substance by the permeation amount of antipyrine is shown as an in vitro relative permeation amount in Table 5. A relative permeation amount of each test substance obtained from the permeability test by the placenta perfusion method using term placenta was excerpted from "Li, H. et al. Arch. Toxicol. 2013, 87, 1661-1669" and shown as an ex vivo relative permeation amount (literature value) in Table 5.

**[Table 5]**

| Name of test substance | In vitro relative permeation amount | Ex vivo relative permeation amount (literature value) |
|---|---|---|
| Antipyrine | 1 | 1 |
| Caffeine | 1.1 | 1.5 |
| Indomethacin | 0.62 | 0.66 |
| Ofloxacin | 0.31 | 0.34 |

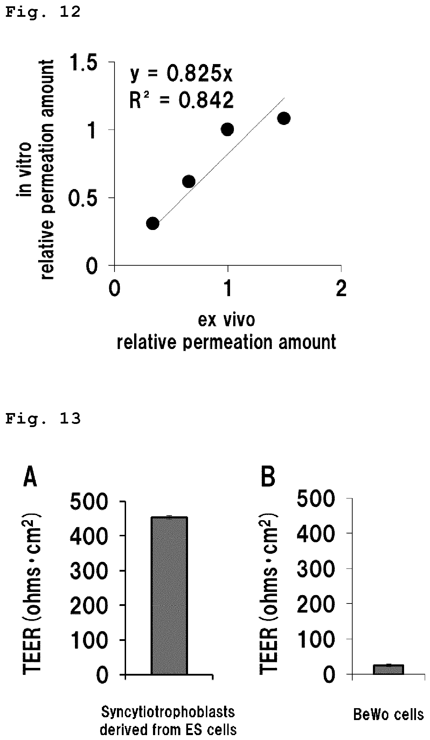

Correlation between the in vitro relative permeation amount and ex vivo relative permeation amount of each test substance was examined. The result is shown in Fig. 12. High correlation was shown as seen from the square of the correlation coefficient between the in vitro relative permeation amount and ex vivo relative permeation amount of each test substance of 0.842 (Fig. 12).

This result revealed that the cell layer permeability of a test substance in placenta cells can be assayed by using the cells produced from human iPS cells by Production method of the present invention.

### Example 16: Transepithelial resistance of syncytiotrophoblasts produced from human ES cells and human choriocarcinoma cell line

### (1) Production of syncytiotrophoblasts using human ES cells, and measurement of transepithelial resistance

KhES-1 cells were differentiated into trophoblasts according to the method described in Example 13, and seeded again on a cell culture insert, and cultured for two days at 37°C, 5% CO₂. Culturing of six wells was conducted in the same condition.

After two days from the seeding to the cell culture insert, the electric resistance (Ω) of the cell layer formed on the cell culture insert was measured by using a Millicell ERS-2 (Millipore), and from the obtained measurement value, the electric resistance of the blank in which cells are not seeded was subtracted, and the resultant value was multiplied with the area of the membrane filter (cm²) to determine a transepithelial electric resistance (TEER, Ω•cm²). The result is shown in Fig. 13A. In Fig. 13A, a standard deviation of TEER value is indicated by an error bar. Syncytiotrophoblasts derived from human ES cells showed a resistance value of as high as 453.

### (2) Culturing of human choriocarcinoma cell line and measurement of transepithelial resistance

Human choriocarcinoma cell line BeWo cells (available from Japan Health Science Foundation) were seeded on a 100 mm cell culture dish, and cultured at 37°C, 5% CO₂. Culturing of six wells was conducted in the same condition. As a culture medium at that time, a culture medium in which 10% FBS (Corning), 2 mM L-glutamine, and 100 U/ml penicillin, 100 µg/ml streptomycin (Penicillin-Streptomycin Mixed Solution, Nacalai Tesque) are added to a DMEM/F12 medium (Sigma-Aldrich)(hereinafter, referred to as BeWo medium) was used.

The BeWo cells were washed twice with a phosphate-buffered saline (PBS, Invitrogen) while the cells were adhered to the cell culture dish, and then 0.25% trypsin -EDTA solution (Nacalai Tesque) was added to the dish, and the dish was incubated for 2 minutes at 37°C, 5% CO₂. After adding BeWo medium to the dish, cells were peeled off by pipetting and the culture medium containing the cells was collected, and centrifuged (1000 rpm, 3 minutes). After removing the supernatant from the centrifuged culture, the deposit was suspended by adding a BeWo medium. The obtained cells were suspended in 200 µl of culture medium so that 2.5 × 10⁴ cells were contained in one well, and seeded on a cell culture insert coated with collagen I (Cellmatrix type I-C, Nitta Gelatin), and cultured at 37°C, 5% CO₂. The culture medium replacement by BeWo medium was conducted every two days.

On day 14 from the seeding to the cell culture insert, the electric resistance (Ω) of the cell layer formed on the cell culture insert was measured by using a Millicell ERS-2 (Millipore), and from the obtained measurement value, the electric resistance of the blank in which cells are not seeded was subtracted, and the resultant value was multiplied with the area of the membrane filter (cm²) to determine a transepithelial electric resistance (TEER, Ω•cm²). The result is shown in Fig. 13B. In Fig. 13B, a standard deviation of TEER value is indicated by an error bar. The TEER value of the BeWo cells was 25, which was a lower resistance value compared with that of the cells produced from human ES cells.

### Example 17: Transporter expression in syncytiotrophoblasts produced from human ES cells and human choriocarcinoma cell line

### (1) Production of syncytiotrophoblasts using human ES cells

The mass of human ES cells having been subjected to the MEF removing operation was dispersed into single cells by using TrypLE Express (Life Technologies), and the obtained cells were suspended in 150 µl of a culture medium so that 1.5 × 10³ cells are contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal medium B (DMEM/F12 medium supplemented with 10% FBS, 2 mM L-glutamine, 100 U/ml penicillin, 100 µg/ml streptomycin, 100 ng/ml BMP4, 300 nM PD 173074) (this point of time is day 0), and the cells were cultured at 37°C, 5% CO₂. After three days from the culture medium replacement, the culture medium was replaced by the differentiation medium having the same composition except that PD 173074 is not contained. On day 4 (day 4) of the culturing using the differentiation medium, cells were washed twice with PBS, and then fixed with 4% paraformaldehyde for 15 minutes.

### (2) Culturing of human choriocarcinoma cell line

Human choriocarcinoma cell line BeWo cells (available from Japan Health Science Foundation) were seeded on a 96-well culture plate (Edge plate, Nunc), and cultured at 37°C, 5% CO₂. As the culture medium at that time, a BeWo medium was used. On day 1 after seeding, cells were washed twice with PBS, and then fixed with 4% paraformaldehyde for 15 minutes.

### (3) Cell observation

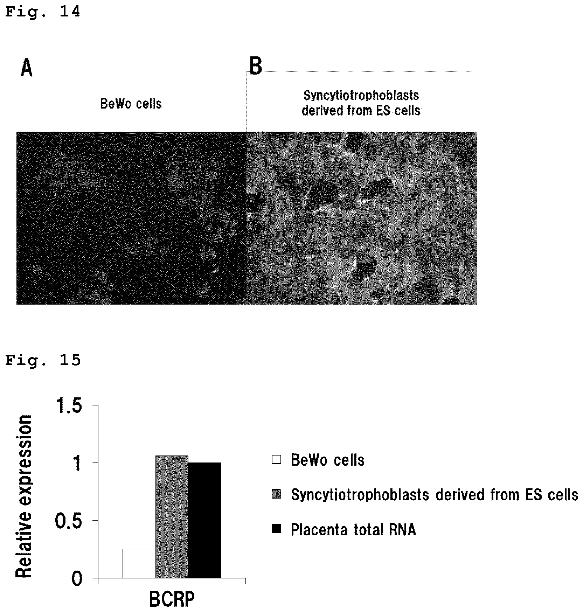

After blocking the cells that were fixed in (1) and (2) for one hour to two hours with 3% BSA, the cells were immunostained by using 4 µg/ml mouse anti-Mdr1 antibody (Santa Cruz Biotechnology), 2 µg/ml donky anti-mouse IgG antibody Alexa 488 (Life technologies) and 1 µg/ml Hoechst 33342 (DOJINDO). The immunostained image of the obtained stained sample was observed under a fluorescence microscope. The result is shown in Fig. 14.

In the BeWo cells, green fluorescence that is indicative of expression of a drug efflux transporter Mdr1 gene that is known to be expressed in human syncytiotrophoblasts was not observed (Fig. 14A). On the other hand, in syncytiotrophoblasts derived from human ES cells, cells of about 80% showed green fluorescence (see Fig. 14B).

### Example 18: Transporter expression in syncytiotrophoblasts produced from human ES cells and human choriocarcinoma cell line

### (1) Production of syncytiotrophoblasts using human ES cells

The mass of human ES cells having been subjected to the MEF removing operation was dispersed into single cells by using TrypLE Express (Life Technologies), and the obtained cells were suspended in 150 µl of a culture medium so that 1.5 × 10³ cells are contained per one well, and seeded on a 96-well culture plate (Edge plate, Nunc) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal medium B (DMEM/F12 medium supplemented with 10% FBS, 2 mM L-glutamine, 100 U/ml penicillin, 100 µg/ml streptomycin, 100 ng/ml BMP4, 300 nM PD 173074) (this point of time is day 0), and the cells were cultured at 37°C, 5% CO₂. After three days from the culture medium replacement, the culture medium was replaced by the differentiation medium having the same composition except that PD 173074 is not contained, and the cells were further cultured for two days. On day 5, total RNA was extracted from cells by using RNeasy Micro Kit (QIAGEN).

### (2) Culturing of human choriocarcinoma cell line

Human choriocarcinoma cell line BeWo cells (available from Japan Health Science Foundation) were seeded on a 100 mm cell culture dish, and cultured at 37°C, 5% CO₂. As the culture medium at that time, a BeWo medium was used. On day 3 after seeding, total RNA was extracted from cells using RNeasy Micro Kit (QIAGEN).

### (3) Gene expression analysis

The total RNA obtained in (1) and (2), and Human Placenta Total RNA (TAKARA BIO) were reverse transcribed by using SuperScript III (Invitrogen), and by real-time PCR using the obtained DNA as a template, TaqMan probe (Applied biosystems) and TaqMan Fast advanced master mix (Applied biosystems), the expression amount of BCRP gene which is a drug efflux transporter was examined. For the cells obtained in each culturing, the mRNA expression amounts of BCRP gene and GAPDH gene which is a house keeping gene were determined by real-time PCR. The value obtained by dividing the mRNA amount of BCRP gene by the mRNA amount of GAPDH gene is defined as a correction value of mRNA amount of BCRP gene, and taking the BCRP gene mRNA amount correction value in Human Placenta Total RNA as 1, a BCRP gene mRNA amount correction value in syncytiotrophoblasts derived from human ES cells or in BeWo cells was shown as a relative expression amount. The result is shown in Fig. 15. In Fig. 15, the relative expression amount of BeWo cells is indicated by a white bar graph, the relative expression amount of syncytiotrophoblasts derived from human ES cells is indicated by a gray bar graph, and the relative expression amount of Human Placenta Total RNA is indicated by a black bar graph.

In the BeWo cells, the BCRP gene mRNA expression amount was about 0.25 times the BCRP gene mRNA expression amount in Human Placenta Total RNA. On the other hand, in the cells obtained in Production example of the present invention, the BCRP gene mRNA expression amount was about 1.06 times the BCRP gene mRNA expression amount in Human Placenta Total RNA.

### Example 19: Drug efflux transporter function analysis in syncytiotrophoblasts produced from human ES cells, and human choriocarcinoma cell line

### (1) Production of syncytiotrophoblasts using human ES cells

The mass of human ES cells having been subjected to the MEF removing operation was dispersed into single cells by using TrypLE Express (Life Technologies), and the obtained cells were suspended in 1.5 ml of a culture medium so that 2.5 × 10⁴ cells are contained per one well, and seeded on a 6-well culture plate (BD Falcon) coated with 20-fold diluted Matrigel (Growth Factor Reduced, BD Biosciences), and maintenance cultured at 37°C, 2% CO₂. For maintenance culturing, a culture medium prepared by adding 10 ng/ml bFGF and 20 µM Y-27632 to MEF-CM was used.

After two days from the start of the maintenance culturing, the culture medium was replaced by a differentiation medium prepared by adding 0.1 µM S26948 (PPAPγ agonist), 0.1 µM CGP 7930 (GABABR positive allosteric modulator), 10 nM SR 11237 (RXR agonist), and 1 nM retinoic acid (RAR agonist) to Basal medium B (DMEM/F12 medium supplemented with 10% FBS, 2 mM L-glutamine, 100 U/ml penicillin, 100 µg/ml streptomycin, 100 ng/ml BMP4, 300 nM PD 173074) supplemented with 20µM Y-27632 (this point of time is day 0), and the cells were cultured at 37°C, 5% CO₂. After three days from the culture medium replacement, the culture medium was replaced by the differentiation medium having the same composition except that PD 173074 is not contained, and the cells were further cultured for two days.

### (2) Culturing of human choriocarcinoma cell line

Human choriocarcinoma cell line BeWo cells (available from Japan Health Science Foundation) were seeded on a 6-well culture plate (BD Falcon), and cultured at 37°C, 5% CO₂. As the culture medium at that time, a BeWo medium was used.

### (3) Transporter substrate incorporation experiment

After washing the cells obtained in (1) and (2) once with PBS, HBSS (Life Technologies) supplemented with 0.1% DMSO (Sigma-Aldrich) or 100 µM verapamil (MDR1 inhibitor, Sigma-Aldrich) was added, and the cells were incubated at 37°C, 5% CO₂. The culturing was conducted while using three wells for each condition. After 30 minutes, HBSS was replaced by HBSS having the same composition except that 1 µM calcein AM (MDR1 substrate, DOJINDO) is further added, and the cells were incubated at 37°C, 5% CO₂. After 60 minutes, the cells were washed once with ice-cooled PBS, and then 0.2N NaOH (Nacalai Tesque) containing 0.2% Triton X-100 (Wako) was added, and then the cells were incubated overnight at 37°C, 5% CO₂ to obtain a cell lysate.

### (4) Analysis of transporter function

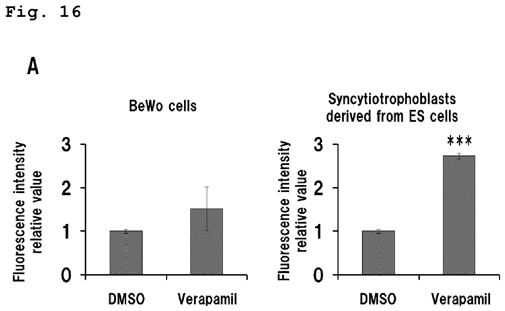

Fluorescence intensity of calcein contained in the cell lysate obtained in (3) was examined by a fluorescence plate reader. The protein concentration of the cell lysate obtained in (3) was examined by BCA Protein Assay Kit (Thermo Scientific) . The value obtained by dividing the value of calcein fluorescence intensity of the cell lysate by the value of protein concentration of the cell lysate is defined as a correction value of calcein fluorescence intensity, and taking the correction value of calcein fluorescence intensity in the DMSO added condition as 1, the correction value of calcein fluorescence intensity in syncytiotrophoblasts derived from human ES cells or in BeWo cells was shown as a relative value. The result is shown in Fig. 16. A standard deviation of relative value is indicated by an error bar. The fluorescence intensity relative value having a statistically significant difference with respect to the fluorescence intensity relative value in the DMSO added example is marked with asterisks (*** means a P value of less than 0.001). In the BeWo cells, the fluorescence intensity relative value increased about 1.5 times in the verapamil added example, compared with the DMSO added example, however, the variation was not significant (Fig. 16A). In the syncytiotrophoblasts derived from human ES cells, the fluorescence intensity relative value significantly increased about 2.7 times in the verapamil added example, compared with the DMSO added example (Fig. 16B).

Non-fluorescent calcein AM easily permeates cellular membrane, and is hydrolyzed in cytoplasm by intracellular esterase into calcein that is membrane-impermeable and shows green fluorescence. Calcein is a MDR1 substrate, and is effluxed from cells by MDR1. Since increase in intracellular calcein concentration by verapamil which is a MDR1 inhibitor means expression of functional MDR1 on the cellular membrane, Example 17 suggested that the syncytiotrophoblasts derived from human ES cells had a larger quantity of functional transporter than the human choriocarcinoma cell line.

The results of Examples 14 to 17 suggested that by using the cells produced from human ES cells, it is possible to assay the placenta cell layer permeability of a test substance more accurately in comparison with using a human choriocarcinoma cell line.

### INDUSTRIAL APPLICABILITY

According to Production method of the present invention, it becomes possible to produce artificial syncytiotrophoblasts derived from human cells, and progenitor cells thereof in vitro with high efficiency. The artificial syncytiotrophoblasts and progenitor cells thereof produced by Production method of the present invention can be utilized for evaluating the toxicity or the drug efficacy of a chemical substance or the like, and for analyzing clinical condition.

## Claims

1. A method for producing artificial trophoblasts derived from human cells, comprising the steps of: adhesion culturing human pluripotent stem cells in a culture medium containing a BMP signal transduction activator, and during the culturing bringing the cells under culturing into contact with at least one selected from the group consisting of a γ aminobutyric acid B receptor activator, a peroxisome proliferator-activated receptor γ activator, a retinoid X receptor activator, and a retinoic acid receptor activator, thereby obtaining a culture containing trophoblasts differentiated from the human pluripotent stem cells.

2. A method for producing artificial syncytiotrophoblasts derived from human cells, comprising the steps of: adhesion culturing human pluripotent stem cells in a culture medium containing a BMP signal transduction activator, and during the culturing bringing the cells under culturing into contact with at least one selected from the group consisting of a γ aminobutyric acid B receptor activator, a peroxisome proliferator-activated receptor γ activator, a retinoid X receptor activator, and a retinoic acid receptor activator, thereby obtaining a culture containing syncytiotrophoblasts differentiated from the human pluripotent stem cells.

3. The production method according to claim 1 or 2, wherein the contact of the cells under culturing with at least one selected from the group consisting of a γ aminobutyric acid B receptor activator, a peroxisome proliferator-activated receptor γ activator, a retinoid X receptor activator, and a retinoic acid receptor activator is started before cells expressing GCM1 mRNA appear in the culture.

4. The production method according to any one of claims 1 to 3, wherein the contact of the cells under culturing with at least one selected from the group consisting of a γ aminobutyric acid B receptor activator, a peroxisome proliferator-activated receptor γ activator, a retinoid X receptor activator, and a retinoic acid receptor activator is conducted from before cells expressing GCM1 mRNA appear in the culture to after cells expressing GCM1 mRNA appear.

5. The production method according to any one of claims 1 to 4, wherein the contact of the cells under culturing with at least one selected from the group consisting of a γ aminobutyric acid B receptor activator, a peroxisome proliferator-activated receptor γ activator, a retinoid X receptor activator, and a retinoic acid receptor activator is conducted over the entire period of the culturing in the culture medium containing a BMP signal transduction activator.

6. The production method according to any one of claims 1 to 5, wherein the culture medium containing a BMP signal transduction activator is a culture medium that contains a BMP signal transduction activator but is free of an FGF signal transduction activator.

7. The production method according to any one of claims 1 to 6, wherein the BMP signal transduction activator is Bone Morphogenetic Protein 4.

8. The production method according to any one of claims 1 to 7, wherein the cells under culturing is further brought into contact with an FGF signal transduction inhibitor before cells expressing GCM1 mRNA appear in the culture.

9. The production method according to any one of claims 1 to 8, wherein at least one selected from the group consisting of a γ aminobutyric acid B receptor activator, a peroxisome proliferator-activated receptor γ activator, a retinoid X receptor activator, and a retinoic acid receptor activator are a γ aminobutyric acid B receptor activator, and at least one selected from the group consisting of a peroxisome proliferator-activated receptor γ activator, a retinoid X receptor activator, and a retinoic acid receptor activator.

10. The production method according to any one of claims 1 to 9, wherein at least one selected from the group consisting of a γ aminobutyric acid B receptor activator, a peroxisome proliferator-activated receptor γ activator, a retinoid X receptor activator, and a retinoic acid receptor activator are a γ aminobutyric acid B receptor activator, a peroxisome proliferator-activated receptor γ activator, a retinoid X receptor activator, and a retinoic acid receptor activator.

11. The production method according to any one of claims 1 to 10, wherein the human pluripotent stem cells are human pluripotent stem cells that are maintenance cultured after singly dispersed.

12. The production method according to any one of claims 1 to 11, wherein the pluripotent stem cells are embryonic stem cells or induced pluripotent stem cells.

13. Artificial trophoblasts or artificial syncytiotrophoblasts produced by the method according to any one of claims 1 to 12.

14. A kit comprising artificial trophoblasts or artificial syncytiotrophoblasts produced by the method according to any one of claims 1 to 12.

15. A method for assaying cell layer permeability of a test substance, comprising: bringing the test substance into contact with artificial trophoblasts or artificial syncytiotrophoblasts produced by the method according to any one of claims 1 to 12, and assaying permeability of the substance to the trophoblasts or syncytiotrophoblasts.

16. Use of artificial trophoblasts or artificial syncytiotrophoblasts produced by the method according to any one of claims 1 to 12 as a reagent for evaluating toxicity or drug efficacy.

17. A method for evaluating toxicity or drug efficacy of a test substance, comprising bringing the test substance into contact with artificial trophoblasts or artificial syncytiotrophoblasts produced by the method according to any one of claims 1 to 12, and assaying the influence of the substance on the trophoblasts or syncytiotrophoblasts.

18. A method for analyzing clinical condition of a disease due to a damage of placenta tissue, comprising, by using artificial trophoblasts or artificial syncytiotrophoblasts produced by the method according to any one of claims 1 to 12, examining substance transportation or hormone secretion to which the trophoblasts or syncytiotrophoblasts are involved.

19. Use of artificial trophoblasts or artificial syncytiotrophoblasts produced by the method according to any one of claims 1 to 12 as a reagent for analyzing clinical condition of a disease due to a damage of placenta tissue.
